# EUROPÄISCHE PATENTSCHRIFT

(11) **EP 2 614 840 B1**
(45) Veröffentlichungstag und Bekanntmachung des Hinweises auf die Patenterteilung: **28.11.2018**
(21) Anmeldenummer: 12199382.8
(22) Anmeldetag: 27.12.2012
(51) Int. Cl.: A61L 2/26, A61L 2/07

(54) **Behälter für medizinische oder zahnmedizinische Instrumente zum Anbringen in einem Autoklaven**
Container for medical or dental instruments for fitting in an autoclave
Récipient pour instruments médicaux ou dentaires destiné à la réception dans un autoclave

(30) Priorität: 11.01.2012 DE 102012200331
(43) Veröffentlichungstag der Anmeldung: 17.07.2013
(73) Patentinhaber: MELAG Medizintechnik oHG, 10829 Berlin (DE)
(72) Erfinder: Creutzburg, Kai-Hendrik, 12487 Berlin (DE); Podolski, Denis, 13585 Berlin (DE); Bonatz, Stefan, 12623 Berlin (DE); Hartrotdt, Matthias, 16341 Panketal (DE); Seibert, Philipp, 13409 Berlin (DE)
(74) Vertreter: Maikowski & Ninnemann Patentanwälte Partnerschaft mbB

(56) Entgegenhaltungen:
- EP-A1- 1 477 106
- EP-A1- 2 356 952
- EP-A1- 2 606 913
- WO-A1-2004/030707
- WO-A1-2009/039616
- WO-A1-2011/101214
- WO-A2-2004/043499
- WO-A2-2005/105161
- WO-A2-2007/103278
- DE-A1-102009 011 147
- DE-U1-202004 006 723
- US-A- 4 296 862
- STORZ, W.; GABELE, L.: "MicroStop Sterilbarriere", , 31. Dezember 2001 (2001-12-31), XP002696464, Gefunden im Internet: URL:http://www.klsmartin.com/fileadmin/Inh alte/Downloads_Prospekte/Sterilcontainer/Z entralsterilisation.pdf [gefunden am 2013-05-03]

## Beschreibung

Die vorliegende Erfindung betrifft einen Behälter für medizinische oder zahnmedizinische Instrumente gemäß dem Obergriff des Anspruchs 1.

EP1477106 A1 offenbart einen Sterilisierbehälter zum Reinigen und Autoklavieren von Endoskopen mit Anschlüssen zur Behandlung der Endoskopkanäle. Es ist bekannt, die hygienische Aufbereitung von medizinischen oder zahnmedizinischen Instrumenten in Autoklaven durchzuführen. Autoklaven arbeiten üblicherweise mit Wasserdampf und unter hohem Druck, um eine gewünschte Sterilisation bzw. Desinfektion und damit eine hygienische Aufbereitung von verwendeten medizinischen oder zahnmedizinischen Instrumenten zur erreichen. Es ist ebenfalls bekannt, den Schritt der Sterilisation mit der Reinigung der medizinischen und zahnmedizinischen Instrumente unter Verwendung geeigneten Prozessmedien bzw. Chemikalien durchzuführen.

Zur Reinigung von zahnmedizinischen Instrumenten, wie zum Beispiel Bohrer, Haken, Absaugstutzen und Ähnliches ist es ebenfalls bekannt, diese zahnmedizinischen Instrumente in separaten, in den Autoklaven einbringbaren Kassetten bzw. Behältern zu lagern. Solche Kassetten bzw. Behälter zur Aufbereitung von medizinischen oder zahnmedizinischen Instrumenten sind zum Beispiel aus der EP 870512 B1 und EP 638298 B1 bekannt.

In der EP 870512 B1 wird zum Beispiel die Verwendung von Kassetten beschrieben, in denen Adapter zur Aufnahme von medizinischen oder zahnmedizinischen Instrumenten angeordnet sind. Die in den Adaptern angeordneten Instrumente weisen in der Regel Hohlräume auf, welche durch eine geeignete Flüssigkeitszufuhr durch die Adapter und im folgenden durch die Hohlräume der eingeführten Instrumente mit Reinigungsflüssigkeit in adäquater Weise gereinigt werden können. Nachteilig bei dem in den genannten Druckschriften beschriebenen Kassetten ist, dass die beschriebenen Kassetten lediglich in speziell für diese Kassetten ausgelegten Geräten, das heißt für die Kassetten spezifisch konstruierten Autoklaven, verwendet werden können.

Nachteilig ist in diesen Konstruktionen auch, dass die die Aufbereitungskassette umgebende Aufbereitungskammer nicht vor Verschmutzungen, wie etwa durch die Reinigungsflüssigkeit oder Pflegeflüssigkeit, geschützt wird. Dies grenzt die Nutzbarkeit der Geräte zur Aufbereitung, insbesondere zur Sterilisation von Standardinstrumenten in größeren Umfang stark ein, so dass die aus dem Stand bekannten Kassetten im Rahmen eines Standardautoklaven nicht einsetzbar sind.

Zur Vermeidung des Verschmutzungsproblems wird in der WO 2006/126103 A2 ein weitergehender Ansatz beschrieben. So wird hier ein Autoklav vorgestellt, der mittels eines in den Autoklaven einführbaren zusätzlichen Behälters zur Aufbereitung von zum Beispiel zahnmedizinischen Instrumenten umgerüstet werden kann. Der beschriebene Instrumentenbehälter ist dabei als selbsttragender Druckbehälter im Sinne einer Autoklavenkammer ausgelegt, wodurch eine hermetische Abriegelung zwischen der Autoklavenkammer und dem zusätzlichen Behälter erfolgen kann. Diese Ausgestaltung bietet den Vorteil, dass durch etwaige Verschmutzungen an den medizinischen und zahnmedizinischen Instrumenten, die im Verlauf der Reinigung und Sterilisation entfernt werden, sowie durch eventuelle Pflegemittelreste keine Kontamination des gesamten Autoklaveninnenraums erfolgt, so dass der Autoklav uneingeschränkt zur Aufbereitung von anderen Standardinstrumenten einsetzbar ist.

Die Umsetzung eines solchen, separat im Autoklaveninnenraum anbringbaren Instrumentenbehälters setzt allerdings eine entsprechend massive und stabile Konstruktion des Behälters voraus. Diese erforderliche massive Konstruktion des Behälters erfordert allerdings verlängerte Aufheizzeiten, wodurch etwaige Energie- und Zeitvorteile eines in der WO 2006/126103 A2 beschriebenen Systems durch die verlängerten Aufheizzeiten teilweise kompensiert werden. Zusätzlich ist für eine derart druckfeste Ausgestaltung eines Instrumentenbehälters ein höherer Fertigungsaufwand notwendig. Des Weiteren ist es erforderlich, neben den Prozessmedien, wie Reinigungsflüssigkeiten oder Pflegeflüssigkeiten, ebenfalls die entsprechenden Mittel zur Prozessüberwachung in den beschriebenen Instrumentenbehälter einzuführen, was den Konstruktionsaufwand weiter erhöht, insbesondere dann, wenn der Instrumentenbehälter ebenfalls als Sterilbehälter benutzt werden soll.

Aus der DE 19913417 ist ein Sterilisierbehälter bekannt, der den Transport des Sterilisationsmediums über ein System von Filtern und Ventilen gewährleistet. Die Ventilanordnung ermöglichst den Medienaustausch innerhalb eines Sterilisators bis zur Vakuum-Trockenphase und schließt erst in der letzten Belüftungsphase, so dass der in der Belüftungsphase herrschende Unterdruck in dem Sterilbehälter aufrechterhalten bleibt und eine Vakuumversiegelung des Sterilbehälters gewährleistet wird. Der von dem Autoklaveninnenraum getrennte Behälter ist allerdings nicht zur vollständigen Aufbereitung von ärztlichem, insbesondere zahnärztlichem Instrumentarium geeignet. Durch die, bei der Instrumentenaufbereitung immanent auftretenden, Spritzer, Sprühnebel und Aerosole wäre eine Anordnung, wie sie aus DE 19913417 bekannt ist, einer ständigen Verschmutzung ausgesetzt und würden ein erhebliches Hygienerisiko darstellen, weil sich die Verunreinigungen in den Filtern und Ventilen anreichern und sich diese so zu einem Kontaminationsherd entwickeln.

Der vorliegenden Erfindung liegt daher die Aufgabe zu Grunde, die aus dem Stand der Technik bekannten Nachteile zu überwinden und einen Behälter zur Aufnahme von medizinischen oder zahnmedizinischen Instrumenten zum lösbaren Anbringen in einem Autoklaven bereitzustellen, der eine hygienische Aufbereitung der Instrumente ohne Kontamination bzw. Verschmutzung des Autoklaveninnenraums und des Instrumentenbehälters selbst ermöglicht, ohne dabei druckfest, d.h. druckfest im Sinne einer zur Sattdampfsterilisation ausgelegten Autoklavenkammer, ausgestaltet sein zu müssen.

Erfindungsgemäß wird diese Aufgabe mit einem Behälter nach Anspruch 1 gelöst.

Entsprechend wird ein Behälter zur Aufbereitung von medizinischen oder zahnmedizinischen Instrumenten in einem Autoklaven und zum lösbaren Anbringen in einem Innenraum eines Autoklaven bereitgestellt, wobei der Behälter mindestens ein erstes Mittel zur Zufuhr von mindestens einem Sterilisationsmedium aus dem Innenraum des Autoklaven, in dem der Behälter angebracht ist, in den Behälter umfasst. Dabei ist das mindestens eine Mittel zur Zufuhr des Sterilisationsmediums in den Behälter als labyrinthförmige Leitung, als mäanderförmige Leitung und/oder als mäanderförmiges Bauteil ausgebildet. Auch umfasst der Behälter mindestens ein zweites Mittel zur Zufuhr von Flüssigkeiten zur äußeren und inneren Reinigung der medizinischen oder zahnmedizinischen Instrumente in den Behälter, und mindestens ein drittes Mittel zur Abfuhr der Flüssigkeiten zur äußeren und inneren Reinigung der medizinischen oder zahnmedizinischen Instrumente und/oder dem Sterilisationsmedium aus den Behälter . Zudem ist der vorliegende Behälter als nicht selbsttragend gegenüber Prozessdrücken ausgelegt.

Der erfindungsgemäß vorgesehene Behälter ist somit als offenes System ausgelegt, das mit der Innenkammer des Autoklaven in Verbindung steht, wobei jedoch eine Kontamination des Autoklaveninnenraums durch eventuell aus dem Behälter austretende Flüssigkeit, zum Beispiel in Form von Flüssigkeitsspritzern, vermieden wird.

Kerngedanke des vorliegenden Behälters ist es, einen hermetisch abgeriegelten Behälter im Sinne einer Reinigung und Pflege von Übertragungsinstrumenten zu gestalten, der jedoch im Sinne einer Desinfektion und/oder Sterilisation ein offenes System darstellt.

Werden nun die Reinigungs- und Pflegemittel in den Behälter eingebracht, so können diese Aufbereitungsprozesse stattfinden, ohne dass diese Medien in die Autoklavenkammer austreten. Soll eine Desinfektion und/oder Sterilisation stattfinden, kann das Sterilisationsmedium in den Behälter eindringen und seine Wirkung entfalten.

Das Mittel zur Zufuhr eines Sterilisationsmediums in Form einer dafür geeigneten Leitung stellt eine Verbindung und somit auch einen Druckausgleich zwischen dem Behälter und der Autoklavenkammer her. Diese Leitung kann erfindungsgemäß von den Sterilisationsmedien passiert werden, ist jedoch für Reinigungs- und Pflegemittel nicht passierbar. Somit ist eine Kontamination der Kammer durch das Bereitstellen der Leitung für das Sterilsiationsmedium ausgeschlossen, ohne dass der Instrumentenbehälter druckfest ausgelegt werden muss. Gleichzeitig stellt eine solche Leitung für das Sterilsiationsmedium eine technologisch einfache Lösung für den Schutz von Filtern und dergleichen dar, was die hygienische Sicherheit erhöht und die kostengünstige Umsetzung eines erfindungsgemäßen Systems möglich macht.

Der Behälter muss aufgrund seines offenen Systems im Innenraum des Autoklaven positioniert werden, um so die Versorgung mit den entsprechenden Sterilisationsmedien, wie zum Beispiel Sattdampf zu gewährleisten. Die grundsätzliche Idee der vorliegenden Erfindung ist also die Ausführung eines Instrumentenbehälters als offenes System, wobei eine Kontamination mit Pflegemittelresten oder verschmutzten Reinigungsmitteln durch die erfindungsgemäße Gestaltung des Instrumentenbehälters in Kombination mit einer optimierten Prozesssteuerung und eines optimierten Fluidmanagements, insbesondere Flüssigkeitsmanagements verhindert wird.

Wie oben angeführt ist der vorliegende Behälter nicht-druckfest, d.h. nicht selbsttragend gegenüber den Prozessdrücken einer Sattdampfsterilisation, ausgelegt. Mit anderen Worten, der vorliegende Behälter ist nicht dazu vorgesehen oder geeignet, mit einem hohen Innendruck oder Außendruck beaufschlagt zu werden, und ist somit nicht geeignet, zu Sterilisationszwecken separat vom Autoklaveninnenraum verwendet zu werden.

Der Instrumentenbehälter ist bevorzugt in Form eines regelmäßigen Hexaeders mit einer Oberseite und einer Unterseite und vier Seitenflächen ausgebildet, wobei das mindestens eine Mittel zur Zufuhr des Sterilisationsmediums bevorzugt an mindestens einer der Seitenflächen und/oder an der Oberseite des Behälters angeordnet ist.

Das mindestens eine Mittel zur Zufuhr des Sterilisationsmediums des Behälters steht mit dem Innenraum des Autoklaven in einer direkten Fluidverbindung, d.h. das Mittel zur Zufuhr des Sterilisationsmediums führt direkt in den Innenraum des Autoklaven bzw. ragt in den Autoklaveninnenraum hinein, so dass ein direkter Fluidaustausch zwischen Autoklaveninnenraum und Behälter ermöglicht wird.

Zusätzlich ist auch möglich, dass mindestens ein weiteres eine Mittel zur Zufuhr des Sterilisationsmediums vorgesehen ist, wobei das Sterilisationsmedium vom Behälter aus den Autoklaveninnenraum zunächst hinausgeführt und wieder in den Autoklaveninnenraum in Form eines Bypass, insbesondere als Bypass mit Ventil, zurückgeführt wird.

Eine labyrinthförmige Gestaltung der Zufuhrleitung für das Sterilisationsmedium basiert auf dem Konzept eines Labyrinthfilters, welches dem Fachmann bekannt ist. Die Zufuhrleitung für das Sterilisationsmedium verfügt so über mindestens ein, vorzugsweise aber mehrere Windungen, die bevorzugt einen stetig steigenden Verlauf, relativ zur Horizontalen, nehmen. Die Windungen erhöhen den Widerstand der Leitung gegenüber Fluiden. Werden Spritzer bereits durch eine einzige Windung aufgehalten, erschweren weitere Windungen den Durchgang von Fluiden, abhängig von deren Viskosität. Da beispielsweise Ölnebel in diesem Zusammenhang eine geringere Durchdringungswirkung als Wasserdampf haben, ist es möglich, eine Anzahl von Windungen zu definieren, bei der Ölnebel die Leitung nicht mehr durchdringen können, diese jedoch noch durchlässig für den Sterilisationsdampf bleibt. Die stetige Steigung wiederum bewirkt einen Rücktransport des Öls, wobei dieser durch den einströmenden Wasserdampf zusätzlich begünstigt wird. Beim Kondensatablass aus der Aufbereitungskassette würde das ausgewaschene Öl dann aus der Kassette entfernt. Die Zufuhrleitung für das Sterilisationsmedium weist bevorzugt bis zu 10 Windungen, insbesondere 2 bis 8 Windungen auf. Zusätzlich ist es möglich, bei Verwendung einer lamellenförmigen Geometrie für die Zufuhrleitung den Weg aus der Aufbereitungskassette in die Autoklavenkammer stärker zu hemmen als in der Gegenrichtung. Somit ist eine Abgrenzung nicht nur über die Viskosität der verschiedenen Fluide sondern auch über eine geeignete Geometrie möglich.

In einer Variante ist die mäanderförmige Leitung in Form eines Schlauches oder Rohres und/oder in Form eines starren mäanderförmigen Bauteiles an einer Seitenfläche des Behälters und/oder auf der Oberseite des Behälters ausgebildet.

Auch ist eine Kombination einer mäanderförmigen Leitung mit z.B. lediglich ein bis zwei Windungen mit mindestens einem Filter und/oder mit mindesten einem Ventil denkbar. Auf diese Weise kann die Anzahl der Windungen der Leitung deutlich reduziert werden, da diese nur noch einen Schutz des Filters vor kontaminierten Verunreinigungen aus dem Instrumentenbehälter bewirken müssen. Auftretende Ölnebel würden vom Filter aufgefangen. Dieser wiederum würde ebenfalls durch den Sterilisationsdampf ausgewaschen und regeneriert. Der Kondensatablass würde auch hier den Abtransport des aufgefangenen Öls einschließen.

Bei Ausführung des Zufuhrmittels für das Sterilisationsmedium als starres mäanderförmiges Bauteil kann dieses entweder als separates Teil ausgebildet sein, das in die Oberseite oder in eine der seitlichen Wände bzw. Seitenflächen des Instrumentenbehälters einbringbar ist, oder es kann einstückig mit dem Instrumentenbehälter ausgeführt sein.

Bei Ausführung des mäanderförmigen Teils als separates Teil liegt dieses bevorzugt als eine Konstruktion vor, die aus mindestens einem Materialteil besteht, das parallel zur Seitenfläche oder zur Oberseite des Behälters angeordnet ist. In dieser Ausführungsform ist das manderförmige Bauteil bevorzugt so gestaltet, dass das eine Materialteil parallel zu einer Öffnung, die in einer Wand bzw. Seite des Behälters, z.B. Seitenwand oder Oberseite des Behälters vorgesehen ist, angeordnet ist, und zwar so, dass das Materialteil die Öffnung jenseits derselbigen, d.h. über die Öffnung hinaus, verdeckt ohne allerdings einen Zugang des Sterilisationsmediums durch die Öffnung in den Behälterinnenraum zu verhindern. Vielmehr ist das eine Materialteil mit einem Abstand zu der Behälterwand angeordnet, wobei der Abstand z.B. durch die spezifische Form des Materialteils wie z.B. angewinkelte oder abgebogene Form einstellbar ist. Es ist denkbar, dass in der Öffnung selbst ein Filter vorgesehen ist, um z.B. auftretende Ölnebel aufzufangen.

Das mäanderförmige Bauteil kann auch in Form einer Konstruktion aus zwei Materialteilen vorliegen, die zueinander parallel versetzt mit einem vorbestimmten Abstand angeordnet sind. Der vorbestimmte, durch ein Abstandstück z.B. in Form eines Filters eingestellte Abstand zwischen den zwei Materialteilen ermöglicht einen mäanderförmigen Zugang für ein Fluid, wie zum Beispiel Sattdampf in den Instrumentenbehälter.

Wie erwähnt kann die mäanderförmige Konstruktion einstückig mit dem Instrumentenbehälter, insbesondere der Oberseite des Behälters ausgebildet sein. Um diese einstückige Konstruktion zu ermöglichen, weisen mindestens zwei der Seitenflächen des Instrumentenbehälters unterschiedliche Längen bzw. Höhen ausgehend von der Unterseite des Instrumentenbehälters auf. An den oberen Enden dieser zwei unterschiedlich langen Seitenflächen sind jeweils zwei senkrecht zu den mindestens zwei Seitenflächen angeordnete, horizontal zur Unterseite des Instrumentenbehälters verlaufende und parallel zueinander verlaufende Abschnitte angeordnet, die bevorzugt einstückig mit den Seitenflächen ausgebildet sind. Die senkrecht zu den Seitenflächen verlaufenden Abschnitte weisen jeweils eine kleinere Oberfläche als die Unterseite des Instrumentenbehälters auf, so dass keine komplette oder vollständige Abdeckung der Unterseite des Instrumentenbehälters erfolgt. Der Abstand zwischen den senkrecht zu den Seitenflächen des Instrumentenbehälters verlaufenden Abschnitten wird durch die jeweiligen Längen der Seitenflächen bestimmt. Die senkrecht zu den Seitenflächen verlaufenden Abschnitte weisen also jeweils Bereiche auf, die einen offenen Zugang für Fluide aus dem Autoklaveninnenraum in den Instrumentenbehälter und umgekehrt ermöglichen. Diese offenen Bereiche sind dabei derart versetzt zueinander angeordnet, um eine mäanderförmige Führung des Fluids zu ermöglichen.

Bei Verwendung eines zusätzlichen Filters kann dieser als ein Öl- und wasserabweisender Filter zum Beispiel hergestellt aus Polyestervlies wie Lotex- Filtermatten ausgebildet sein, der einen Lotuseffekt aufweist. Auch ist es möglich, dass dieser Filter als Sterilisationsfilter ausgebildet ist, der für Sterilisationsmedien wie dem Sattdampf durchlässig ist, jedoch Mikroorganismen, Viren und weitere Keime zurückhält. Bei Verwendung eines derartigen Sterilisationsfilters kann der vorliegende Behälter auch nach Abschluss des Sterilisationsvorganges und Entnahme aus der Autoklavenkammer zur sterilen Lagerung der gereinigten medizinischen oder zahnmedizinischen Instrumente verwendet werden. Für diesen Fall wären das Zufuhr- und Abfuhrmittel entsprechend mit geeigneten Verschlüssen zu versehen.

Wie bereits erwähnt, kann das mindestens eine zusätzliche Zufuhrmittel für das Sterilisationsmedium in Form eines Bypasses ausgebildet sein. In dieser Variante kann eine Leitung aus dem Autoklaveninnenraum über die Kupplungsstelle für die Reinigungs- und Pflegemittel in den Behälter hineingeführt werden. Auf diese Weise kann ebenfalls eine Verbindung zwischen der Autoklavenkammer und dem Behälter geschaffen werden.

In einer bevorzugten Ausführung wird diese Bypass-Leitung von einem Ventil, insbesondere einem elektronisch steuerbaren Ventil, unterbrochen. Ist dieses Ventil geschlossen, so ist der Behälter hermetisch abgeriegelt und ein Durchgang von Reinigungsmitteln, Verunreinigungen und/ oder Ölnebeln ist nicht mehr möglich. Dementsprechend wäre das Ventil während der Reinigungs- und Pflegeschritten geschlossen. Für eine Sterilisation kann das Ventil geöffnet werden, wodurch ein Durchtritt des Sterilisationsmediums und ein gleichzeitiger Druckausgleich möglich werden. Auch hier ist es in besonderer Weise bevorzugt, die Leitung mit einem Gefälle hin zum Behälter zu gestalten.

Der vorliegende Behälter verfügt erfindungsgemäß über mindestens ein Mittel zur Flüssigkeitszufuhr in den Behälter. Dieses Zufuhrmittel liegt bevorzugt in Form eines Anschlusses zur Zufuhr von Flüssigkeiten, insbesondere Reinigungsflüssigkeiten und/oder Pflegeflüssigkeiten, für die äußere Reinigung und die innere Reinigung der in den Behälter eingebrachten Instrumente vor. Der vorliegende Behälter kann somit über den Anschluss an eine im Autoklaven vorgesehene Versorgungsleitung für Flüssigkeiten und Fluide, wie Reinigungs- und/oder Pflegemittel gekoppelt sein.

Die in den Behälter eingeführte Flüssigkeit wird bevorzugt über in einem Trägersystem und/oder dem Behälter selbst vorgesehene Kanäle zu den Instrumenten und in die Instrumente geleitet. Dies ermöglicht bevorzugt eine Aufbereitung, das heißt Reinigung und Pflege der Außenseite der Instrumente als auch der Hohlräume bzw. Innenkanäle der Instrumente. Reinigungsdüsen für die Außenreinigung befinden sich bevorzugt im oberen Bereich der Seitenwände des Instrumentenbehälters. Die Innenkanäle bzw. Hohlräume der Instrumente sind bevorzugt aufgeteilt in Spray/Luft- und Antriebskanäle, die entsprechend zumindest teilweise sequenziell gereinigt und gepflegt werden können. Es ist auch grundsätzlich denkbar, dass die Prozessmedien zumindest teilweise im Instrumentenbehälter bereitgestellt werden können und mittels einer angekoppelten Prozesssteuerung, beispielsweise über eine Funk oder Steckverbindung angesteuert werden.

Darüber hinaus umfasst der vorliegende Behälter erfindungsgemäß mindestens ein Mittel zur Abfuhr der Flüssigkeit aus dem Behälter, welches bevorzugt in Form eines Anschluss zur Entleerung des Behälters vorliegt. Dieser mindestens eine Anschluss zur Entleerung des Behälters ist bevorzugterweise im unteren Bereich des Behälters, das heißt im Bodenbereich oder in der Unterseite des selbigen, angeordnet und umfasst bevorzugt ein Anschlussstück zum Ankoppeln einer verschließbaren Entleerungsleitung. Es können jedoch auch andere Mittel zur Entleerung des unteren Bereiches wie zum Beispiel Saugrüssel oder Ähnliches vorgesehen sein. Eine sinnvolle Anwendung einer Entleerungsleitung setzt voraus, dass der untere Bereich des vorliegenden Behälters gegenüber Flüssigkeiten dicht ist, das heißt das die Flüssigkeiten lediglich über die vorgesehene Entleerungsleitung aus dem Instrumentenbehälter abgeführt werden können. Die Entleerungsleitung kann mit einer Entsorgungsleitung für Flüssigkeiten des Autoklaven gekoppelt sein. Es ist aber auch generell vorstellbar, dass der Instrumentenbehälter über einen eigenen Abwassertank verfügt.

Das Abfuhrmittel kann ebenfalls in Form von mindestens einem Abströmventil vorliegen, welches im unteren Bereich des Instrumentenbehälters angeordnet ist und zum Herstellen einer Druckdifferenz zwischen Autoklaveninnenraum und Instrumentenbehälter dienen kann. Dieses Abströmventil kann wie gesagt identisch zum beschriebenen Abfuhrmittel in Form eines Entleerungsanschluss sein oder aber auch davon verschieden sein.

Das mindestens eine Abströmventil ermöglicht das Anlegen einer Druckdifferenz zwischen dem Autoklaven Innenraum und Instrumentenbehälter, wobei der Druck im Autoklaveninnenraum größer ist als im Instrumentenbehälter. Hierfür wird im Autoklaveninnenraum ein Überdruck angelegt und gleichzeitig ist das Abströmventil im Instrumentenbehälter geöffnet, wodurch eine Strömung in Richtung des Instrumentenbehälters erzeugt wird. Diese Strömung kann zum Beispiel aus Sattdampf oder aus der im Autoklaveninnenraum befindlichen Luft bestehen. Die erzeugte Strömung wiederum bildet eine Sperre für in dem Instrumentenbehälter während des Reinigungs- und Pflegevorgangs gegebenenfalls auftretende Aerosole. Diese Aerosole treten insbesondere bei der Pflege von zahnmedizinischen Instrumenten mit ölhaltigen Pflegeflüssigkeiten auf. Durch das bevorzugte Verfahren des Anlegens einer Druckdifferenz zwischen Autoklaveninnenraum und Instrumentenbehälter wird somit die Kontamination des Autoklaveninnenraums mit Aerosolen, insbesondere im Falle der Ausführungsformen des Behälters verhindert, in welchen das druckausgleichende Mittel nicht in Form eines Ventils ausgebildet ist.

In einer Ausführungsform des vorliegenden Behälters ist in den Behälter ein Trägersystem umfassend eine Trägerplatte mit daran ausgebildeten Adaptern zur Aufnahme der medizinischen oder zahnmedizinischen Instrumente lösbar angeordnet. Die Trägerplatte des Trägersystems ist mit Kanälen zum Zuführen und Verteilen von Flüssigkeiten wie Reinigungs- und/oder Pflegeflüssigkeiten, zu den Adaptern ausgebildet. Das Trägersystem ist bevorzugterweise im oberen Bereich des Instrumentenbehälters angebracht. Es ist auch vorstellbar, das die Adapter ohne Trägersystem unmittelbar im oberen Bereich des Instrumentenbehälters am Deckel des Instrumentenbehälters selbst angebracht sind. Bei Verwendung eines Trägersystems aus Trägerplatte und Adaptern ist das Trägersystem aus einem medienstabilen Polymerwerkstoff hergestellt. Trägerplatte und Adapter sind dabei lösbar miteinander verbunden, und die Adapter erstrecken sich senkrecht zur Haupterstreckungsebene der Trägerplatte. Bevorzugterweise weisen die Adapter im eingebauten Zustand des Trägersystems und im geschlossenen Zustand des Instrumentenbehälters im Wesentlichen senkrecht nach unten, so dass auch die in den Instrumentenbehälter eingebrachten medizinischen oder zahnmedizinischen Instrumente im geschlossenen Zustand des Instrumentenbehälters senkrecht nach unten weisen.

In einer weiteren Ausführungsform umfasst der vorliegende Behälter mindestens zwei miteinander schwenkbar verbundene Teile. Der Behälter besteht somit also aus mindestens zwei Teilen, wie zum Beispiel zwei Hälften, die durch mindestens ein Scharnier schwenkbar miteinander verbunden sein können. Der Behälter kann daher in einem oberen Bereich, wie zum Beispiel einem Deckelbereich, und einem unteren Bereich, wie zum Beispiel Bodenbereich unterteilt werden.

Sind das erwähnte Trägersystem aus Trägerplatte und Adapter zum Aufnahme der medizinischen oder zahnmedizinischen Instrumente oder auch die Adapter alleine im oberen Bereich des Behälters, insbesondere am Deckelbereich des Behälters angebracht, dann ist es möglich, dass der Deckelbereich des Behälters im geöffneten Zustand wie ein Tablett zur Bestückung des Instrumentenbehälters dient.

In einer weiteren Ausführungsform weist der Behälter eine Türöffnung auf, die als Beladungsöffnung zum Einbringen der Instrumente einzeln oder auf einem mit Adapter versehenden Trägersystem geeignet ist. Der Randbereich der Beladungsöffnung ist dabei mit einer Dichtung versehen, um das Austreten von Flüssigkeit aus dem Instrumentenbehälter in den Autoklaveninnenraum zu verhindern.

Um einen effektiven Transport der in Instrumentenbehälter enthaltenen Flüssigkeit, insbesondere der sich im Bodenbereich des Instrumentenbehälters befindlichen Flüssigkeit, zum Entleerungsanschluss zu ermöglichen, ist gemäß einer Ausführungsform ein Mittel zum Transport der Flüssigkeit zum Entleerungsanschluss vorgesehen. Dieses Transportmittel kann bevorzugt in Form einer schiefen Ebene oberhalb der Unterseite im Bodenbereich des Behälters zur Bildung eines Gefälles hin zum Entleerungsanschluss konstruiert sein.

Darüber hinaus kann der vorliegende Behälter über mindestens ein Mittel zur Einstellung des Flüssigkeitsstandes der sich im Instrumentenbehälter befindlichen Flüssigkeit verfügen. Dieses Mittel dient insbesondere der Regulierung des Flüssigkeitsstandes der als Auffangmedium im Bodenbereich des Instrumentenbehälters fungierenden Reinigungsflüssigkeit und kann entweder unmittelbar im Instrumentenbehälter oder im Autoklaven zum Beispiel in Form von Dosierpumpen, Dosiervolumina oder anderen Sensoren zum Einstellen des Flüssigkeitspegels angeordnet sein, wobei eine Kombination aus Messturbine und Speisepumpe bevorzugt ist. Eine erfindungsgemäße Ausführung dieses Regulierungsmittels ist jedoch nicht an die Art des Mittels selber gebunden.

Es ist des Weiteren bevorzugt, dass der vorliegende Instrumentenbehälter aus einem Material mit einer Wärme- Festigkeits- Charakteristik von kleiner als 2 ·10⁻⁶ N/s K² hergestellt ist. Die Verwendung eines solchen Materials für den vorliegenden Instrumentenbehälter ist für die Behandlung der Instrumente vor und nach der Reinigung und Sterilisation besonders vorteilhaft. Das bevorzugt verwendete Material verfügt zu einem über eine geringe Wärmeleitfähigkeit, und verfügt zum anderen auch über eine geringe Wärmespeicherkapazität und wirkt somit nicht selbst als Wärmestrahler.

Die Wärme-Festigkeits-Charakteristik bezeichnet dabei das Produkt aus der Wärmespeicherzahl und der Wärmeleitfähigkeit dividiert durch den E-Modul des Materials. Bevorzugte Wärme-Festigkeits-Charakteristiken liegen bei weniger als 1 · 10⁻⁶ N/s·K² und insbesondere bei kleiner als 0,5 · 10⁻⁶ N/s·K², insbesondere kleiner als 0,4 · 10⁻⁶ N/s·K², insbesondere kleiner als 0,3 · 10⁻⁶ N/s·K², insbesondere kleiner als 0,2 · 10⁻⁶ N/s·K² und ganz besonders kleiner als 0,15 · 10⁻⁶ N/s·K². Eine Wärme-Festigkeits-Charakteristik in einem Bereich von 0,05 · 10⁻⁶ N/s·K² bis 2 · 10⁻⁶ N/s·K² ist besonders gut geeignet, wobei die zuvor genannten Werte als bevorzugte Obergrenzen weiter bevorzugter Bereiche anzusehen sind. Ein Material mit einer derartigen Wärme-Festigkeits-Charakteristik weist einerseits die benötigte Festigkeit auf, um für die Aufnahme von medizinischen oder zahnmedizinischen Instrumenten hinreichend stabil zu sein, und andererseits eine geeignete niedrige Wärmespeicherfähigkeit, so dass aufbereitete Instrumente, welche sich noch im Instrumentenbehälter befinden, schnell abkühlen können. Vorzugsweise sind neben dem Instrumentenbehälter selber auch die Trägerplatte und/oder die Adapter und/oder das gesamte Trägersystem aus einem Werkstoff mit einer derartigen Wärme-Festigkeits-Charakteristik gefertigt.

In einer weiteren bevorzugten Ausgestaltung ist der Instrumentenbehälter aus einem Material hergestellt, das einen Quotienten aus Wärmespeicherzahl und Wärmeleitfähigkeit aufweist, der größer als 100 000 s/m², insbesondere größer als 300 000 s/m², insbesondere größer als 500 000 s/m², insbesondere größer als 700 000 s/m², insbesondere größer als 800 000 s/m², insbesondere größer als 900 000 s/m² und ganz besonders größer als 900 000 s/m² ist. Ein Bereich von 100 000 s/m² bis 1 500 000 s/m² ist besonders bevorzugt, wobei die vorgenannten Werte als bevorzugte untere Grenze eines weiter bevorzugten Bereichs anzusehen ist, dessen bevorzugte Obergrenze bei 1 300 000 s/m², insbesondere bei 1 200 000 s/m², 1 100 000 s/m² und ganz besonders bei 1 000 000 s/m² liegt.

Geeignete Materialien zur Herstellung des Instrumentenbehälters und seiner Komponenten sind medienstabile Polymerwerkstoffe. Hierunter fallen beispielsweise bioinerte Kunststoffe, wie etwa Polyetheretherketone (PEEK). Andere medien- und temperaturbeständige Kunststoffe sind jedoch ebenfalls als Materialien für das Trägersystem geeignet. Beispielsweise können Polyphenylensulfon (PPSU) oder Polyetherimid (PEI) als Materialien verwendet werden. Bei der Auswahl eines geeigneten Materiales ist jedoch nicht die chemische Zusammensetzung des Materiales sondern deren physikalischen Eigenschaften ausschlaggebend.

Gegenstand der vorliegenden Erfindung ist auch ein Autoklav geeignet zur hygienischen Aufbereitung medizinischer oder zahnmedizinischer Instrumente, der mindestens einen Behälter zur Aufbereitung von medizinischen oder zahnmedizinischen Instrumenten aufweist, wobei ein Behälter mit den oben beschriebenen Merkmalen bevorzugt ist. Der vorliegende Autoklav ist durch Mittel zum Anschluss und Betreiben des Behälters gekennzeichnet.

So weist der vorliegende Autoklav bevorzugt mindestens einen Anschluss zur Zufuhr von Flüssigkeiten, wie den Prozessmedien aus Reinigungs- und Pflegeflüssigkeiten, in den Instrumentenbehälter und einen Anschluss zur Abfuhr von Flüssigkeiten aus dem Instrumentenbehälter auf. Der mindestens eine Anschluss des Autoklaven zur Zuführung von Flüssigkeiten in den Instrumentenbehälter ermöglicht die Zufuhr von Flüssigkeit für eine äußere und eine innere Reinigung der im Instrumentenbehälter angeordneten Instrumente. Insbesondere bevorzugt ist dabei mindestens ein Anschluss für die Zuführung von Flüssigkeit in den Instrumentenbehälter für eine äußere Reinigung und für eine mindestens teilweise sequenzielle innere Reinigung der im Instrumentenbehälter angeordneten Instrumente, das heißt eine Reinigung der Innenkanäle der Instrumente aufgeteilt in Spray/Luft- und Antriebskanäle.

Es ist insbesondere bevorzugt, wenn zwischen den beschriebenen Flüssigkeitsanschlüssen des Behälters und des Autoklaven eine Verbindungsleitung angeordnet ist, die trennbar ist. Es ist auch vorstellbar, dass die Flüssigkeitsanschlüsse so gestaltet sind, dass diese direkt aneinander oder ineinander eingesetzt oder eingeführt werden können. Wesentlich ist dabei, dass eine Trennung der Anschlüsse voneinander möglich ist, so dass der Behälter aus dem Autoklaven einfach und unproblematisch entnehmbar ist.

In einer weiteren bevorzugten Gestaltung verfügt der vorliegende Autoklav über mindestens ein Mittel zum Erkennen eines in den Autoklaven eingebrachten Instrumentenbehälters. Dies ist insbesondere vorteilhaft, um einen Aufbereitungszyklus der im Instrumentenbehälter angeordneten Instrumente über die Prozesssteuerung nur dann zu starten, wenn dieser Instrumentenbehälter auch tatsächlich im Autoklav angebracht ist.

In einer weiteren Variante umfasst der vorliegende Autoklav mindestens ein Mittel zur Durchführung eines Umwälzkreislaufes zwischen dem unteren Bereich des Behälters und dem oberen Bereich des Behälters. Der Autoklav verfügt also bevorzugt über Mittel zum Umwälzen der Flüssigkeit im Behälter vom unteren Bereich des Behälters durch Abfuhr der Flüssigkeit aus dem Behälter durch das mindestens eine Mittel zur Flüssigkeitsabfuhr hin in den oberen Bereich des Behälters durch Zufuhr der aus dem unteren Bereich des Behälters abgeführten Flüssigkeit durch das mindestens Mittel zur Flüssigkeitszufuhr in den Behälter. Dieses Umwälzmittel realisiert somit eine Umwälzung der sich im unteren Bereich des Behälters, das heißt im Instrumentenbehältersumpf, befindlichen Flüssigkeit zu den Mitteln zur äußeren und inneren Reinigung der Instrumente. Ein geeignetes Mittel zur Durchführung solch eines Umwälzkreislaufes innerhalb des Instrumentenbehälters zwischen unteren Bereich und oberen Bereich des Instrumentenbehälters kann insbesondere in Form einer Umwälzpumpe ausgebildet sein.

In einer Variante weist der vorliegende Autoklav bevorzugt ebenfalls mindestens ein Mittel zur Erzeugung eines vorbestimmten Flüssigkeitsstandes im Instrumentenbehälter auf, das zum Beispiel in Form einer mit einer Messturbine gekoppelten Schwingkolbenpumpe zur Förderung einer vorbestimmten Flüssigkeitsmenge in den Instrumentenbehälter ausgelegt sein kann.

Generell ist es auch vorstellbar, dass das beschriebene Mittel zur Durchführung eines Umwälzkreislaufes im Instrumentenbehälter und das Mittel zur Erzeugung eines vorbestimmten Flüssigkeitsstandes im Instrumentenbehälter miteinander kombiniert sind und zum Beispiel in Form einer dafür geeigneten Umwälzpumpe vorliegen.

Der vorliegende Autoklav ist bevorzugt zur Durchführung einer Sterilisation der Klasse B geeignet.

In einer Variante verfügt der vorliegende Autoklav darüber hinaus über ein Mittel zum Erzeugen einer Druckdifferenz zwischen Autoklaveninnenraum und dem Instrumentenbehälter, um wie oben beschrieben eine Sperre für im Instrumentenbehälter auftretende Aerosole bereitzustellen.

Gegenstand der vorliegenden Erfindung ist ebenfalls ein Verfahren zur hygienischen Aufbereitung von medizinischen oder zahnmedizinischen Instrumenten in einem oben beschriebenen Autoklaven. Das Verfahren umfasst die Schritte:
a) Einbringen von mindestens einem medizinischen oder zahnmedizinischen Instrument in mindestens einen oben beschriebenen Instrumentenbehälter und Einbringen des Instrumentenbehälters in den Innenraum des Autoklaven,
b) gegebenenfalls Evakurierung des Autoklaven nach Anbringen des Instrumentenbehälters auf einem Druck von weniger als 500mbar, bevorzugt weniger als 200mbar, insbesondere bevorzugt weniger als 100mbar für einen vorbestimmten Zeitraum mit sich anschließendem Druckausgleich;
c) Zuführen einer vorbestimmten Menge einer Reinigungsflüssigkeit in den Instrumentenbehälter zur Reinigung des mindestens einen in den Instrumentenbehälter eingebrachten medizinischen oder zahnmedizinischen Instruments;
d) Zuführen einer vorbestimmten Menge an Pflegeflüssigkeit in den Instrumentenbehälter zur Pflege des mindestens einen in den Instrumentenbehälter eingebrachten medizinischen oder zahnmedizinischen Instruments;
e) Ausblasen der überschüssigen Pflegeflüssigkeit mittels Druckluft aus dem mindestens einen medizinischen oder zahnmedizinischen Instrument;
f) Gegebenenfalls Sterilisation bzw. Desinfektion des mindestens einen in den Instrumentenbehälter eingebrachten medizinischen oder zahnmedizinischen Instruments durch Zufuhr von mindestens einem Sterilisations- bzw. Desinfektionsmedium;
g) Gegebenenfalls Evakurierung des Autoklaveninnenraums nach Abschluss der Sterilisation auf einem Druck von weniger als 500mbar, bevorzugt weniger als 200mbar, insbesondere bevorzugt weniger als 100mbar für einen vorbestimmten Zeitraum mit anschließendem Druckausgleich;
h) Entnahme des Instrumentenbehälters aus dem Autoklaveninnenraum und des in den Instrumentenbehälter eingebrachten mindestens einen medizinischen oder zahnmedizinischen Instruments.

Die hygienische Aufbereitung, das heißt Reinigung, Pflege und/oder Desinfektion bzw. Sterilisation der im Instrumentenbehälter eingebrachten Instrumente, wird in an sich bekannter Weise durchgeführt. So werden die entsprechenden Instrumente mit einer geeigneten Reinigungsflüssigkeit gespült, anschließend mit einer Pflegeflüssigkeit, insbesondere einer ölhaltigen Pflegeflüssigkeit beaufschlagt und anschließend mit einem geeigneten Desinfektionsmittel desinfiziert oder mit einem geeigneten Sterilisationsmittel sterilisiert. Als Sterilisations- oder Desinfektionsmittel kommen dabei Chemikalien und/oder gesättigter Wasserdampf in Frage, was einem Fachmann bekannt ist.

In einer bevorzugten Ausführungsform des vorliegenden Verfahrens wird die Schritt c) zugeführte Reinigungsflüssigkeit im unteren Bereich des Behälters mit einem vorbestimmten Flüssigkeitsstand vorgelegt. Die Höhe des Flüssigkeitsstandes wird durch geeignete Mittel zur Regulierung des Flüssigkeitsstandes innerhalb des Instrumentenbehälters eingestellt, wobei das Regulierungsmittel entweder unmittelbar am Instrumentenbehälter selber und/oder am Autoklaven angebracht ist. Die Flüssigkeit, wie zum Beispiel die Reinigungsflüssigkeit wird also mit einem vorbestimmten Pegel im unteren Bereich des Instrumentenbehälters vorgelegt und dient als Trägermedium für ausgeblasenes Öl was im Weiteren noch beschrieben werden wird.

Als Reinigungsflüssigkeit zum Vorlegen im Instrumentenbehälter kommen insbesondere Wasser oder eine aus einer vorhergehenden Instrumentenbehandlung recycelte Reinigungsflüssigkeit in Frage. Um die Fähigkeit des Wassers zur Aufnahme von hydrophoben Substanzen, wie zum Beispiel hydrophoben Substanzen aus der ölhaltigen Pflegeflüssigkeit, zur erhöhen, könnten wenn notwendig waschaktive Substanzen dem Wasser zugefügt werden. Es ist auch möglich, dass die in den Instrumentenbehälter eingeführte Flüssigkeit lediglich zum Zweck der Ölaufnahme aus dem ölhaltigen Pflegemedium in Instrumentenbehälter eingeleitet wird, und nicht zur Reinigung der Instrumente dient.

In einer bevorzugten Variante des vorliegenden Verfahrens wird die in Schritt c) zugeführte Reinigungsflüssigkeit vor und/oder während des Zuführens der Pflegeflüssigkeit in Schritt d) innerhalb des Instrumentenbehälters umgewälzt. Entsprechend erfolgt die Reinigung der Instrumente bevorzugt durch ein Umwälzen der Reinigungsflüssigkeit innerhalb des Instrumentenbehälters. Die Reinigung kann dabei auf verschiedene Temperaturniveaus zum Beispiel mittels kalter Vorreinigung bei Temperaturen unter 45° Grad unter Verwendung einer ersten Reinigungsflotte und mittels Hauptreinigung mit einer erwärmten zweiten Reinigungsflüssigkeit bzw. zweiten Reinigungsflotte durchgeführt werden. Im Anschluss an die Reinigung der Instrumente schließt sich die Instrumentenpflege unter Verwendung einer geeigneten Pflegeflüssigkeit an.

Während der Reinigungs- und Pflegebehandlung erfolgt bevorzugt die Durchführung des Umwälzprozesses der im unteren Behälterbereich aufgefangenen Flüssigkeit während des Durchleitens der ölhaltigen Pflegeflüssigkeit durch die Instrumente zur Steigerung der Effektivität der Ölbindung und somit zur zusätzlichen Vermeidung von Aerosolbildung und Kontamination der Autoklavenkammer. Die zum Umwälzen benötigte Vorrichtung ist insbesondere eine Umwälzpumpe, die im oder am Autoklavengehäuse angeordnet ist. Das Umwälzen beim Pflegen erfolgt bevorzugt über die Düsen zur Außenreinigung.

Wie im vorherigen Absatz angedeutet, wird in dem vorliegenden Verfahren die in Schritt d) zugeführte Pflegeflüssigkeit bevorzugt durch das im Instrumentenbehälter angeordnete mindestens eine medizinische oder zahnmedizinische Instrument durchgeleitet und/oder entlang der Aussenseite der des medizinischen oder zahnmedizinischen Instruments geleitet, wobei die Pflegeflüssigkeit bevorzugt in die Antriebskanäle eingebracht werden. Die Verwendung einer Pflegeflüssigkeit, insbesondere einer ölhaltigen Pflegeflüssigkeit dient dazu, bewegliche Teile der Instrumente vor Verschleiß zu schützen.

In einer besonders bevorzugten Variante des Verfahrens wird, wie oben beschrieben, in dem sich der Reinigung und Pflege anschließenden Schritt e) die sich in den Instrumentenkanälen befindliche überschüssige Pflegeflüssigkeit mittels Druckluft aus dem mindestens einem medizinischen oder zahnmedizinischen Instrument ausgeblasen. Bevorzugterweise wird die in Schritt e) ausgeblasene Pflegeflüssigkeit in dem im unteren Bereich des Instrumentenbehälters zugeführte bzw. vorgelegte Reinigungsflüssigkeit aufgefangen. Die im Instrumentenbehälter vorgelegte Reinigungsflüssigkeit dient sozusagen als Trägermedium für das ausgeblasene, ölhaltige Pflegemittel. Bei einer Entleerung des Instrumentenbehälters kann entsprechend der größte Teil des überschüssigen ölhaltigen Pflegemittels aus dem Instrumentenbehälter, und damit aus dem Autoklaven entfernt werden. Das Auffangen des überschüssigen ölhaltigen Pflegemittels in einer als Trägermedium dienenden Reinigungsflüssigkeit im Bodenbereich des Instrumentenbehälters verhindert somit ein Verölen des Autoklaveninnenraums und gefährdet gleichzeitig nicht die Eigenschaft des Instrumentenbehälters als Sterilbehälter.

Somit ist es also mittels dem vorliegendem Verfahren möglich nach dem Reinigungsschritt und Pflegeschritt sämtliche Verunreinigungen und Pflegemittelreste mit der Reinigungsflüssigkeit aus dem Instrumentenbehälter abzutransportieren. Eine Kontamination der Autoklavenkammer wird somit vermieden. Das Abführen der überschüssiges ölhaltiges Pflegemittel enthaltenden Reinigungsflüssigkeit kann insbesondere durch Verwendung einer Pumpe oder durch Beaufschlagen des Instrumentenbehälters mit Druckluft beschleunigt werden. Somit befinden sich zu Beginn der Sterilisation im Schritt f) lediglich die gereinigten und gepflegten medizinischen oder zahnmedizinischen Instrumente in dem Instrumentenbehälter. Die sich anschließende Sterilisation wird durch Zufuhr eines Sterilisationsmediums durch das dafür vorgesehene Mittel in den Behälter durchgeführt. Als Sterilisationsmedium können entweder geeignete Chemikalien oder bevorzugt erhitzter Wasserdampf (Sattdampf) verwendet werden, der aus dem Autoklaveninnenraum in den Behälter eingeführt wird.

Wie aus dem oben beschriebenen Verfahren des Weiteren entnehmbar, ist ein weiteres optionales Merkmal des vorliegenden Verfahrens die Behandlung der Instrumente vor und nach der Aufbereitung. So hat sich als vorteilhaft erwiesen, dass eine Evakuierung des Autoklaven nach Einbringen des Instrumentenbehälters in den Autoklaven vor Beginn des eigentlichen Reinigungsschrittes c) vom Vorteil ist. Eine derartige Evakuierung vor dem eigentlichen Reinigungsschritt vermindert den Wärmetransport durch Konvektionen und reduziert den Temperaturanstieg im Instrumentenbehälter und somit den Temperaturanstieg der Instrumente. Vor der eigentlichen Reinigung der Instrumente besteht nämlich die Gefahr, dass sich etwaige proteinhaltige Verschmutzungen durch die hohen Temperaturen in der Autoklavenkammer verfestigen. Dies ist insbesondere dann der Fall, wenn die Aufbereitung nicht unverzüglich erfolgt, wobei dieser Effekt in Geräten mit einer Mantelheizung oder einem Doppelmantel verstärkt zu beobachten ist. Eine Evakuierung des Autoklaven vor Beginn der Reinigung und Pflege verringert somit die Gefahr der Verfestigung von Verschmutzungen an den Instrumenten.

Neben einer möglichen Evakuierung der Autoklavenkammer dient auch die Verwendung eines Instrumentenbehälters aus Materialien mit einer Wärme- Festigkeits- Charakteristik von kleiner 2·10⁻⁶N/S · K² wie oben beschrieben einen derartigen Verfestigungseffekt.

Zusätzlich kann es erfindungsgemäß vorgesehen sein, die Instrumente vor der hygienischen Aufbereitung aktiv zu kühlen, um eine unzulässige Überhitzung zu vermeiden. Dies kann beispielsweise durch ein Benetzen der Instrumente im Instrumentenbehälter mit der Reinigungsflüssigkeit erfolgen. In einer weiteren Ausführungsform kann diese Kühlung auch durch Anlegen von Pressluft erfolgen.

Um eine Schädigung der Instrumente nach der Aufbereitung durch hohe Temperaturen vorzubeugen, wird gemäß dem vorliegenden Verfahren vorgeschlagen, den Autoklaveninnenraum nach Durchführung der Reinigungs-, Pflege - und Sterilisationsschritte zu evakuieren, und zwar auf einem Druck von weniger als 500mbar, bevorzugt weniger als 200mbar, insbesondere bevorzugt weniger als 100mbar.

Sämtliche bevorzugte Ausführungsformen des beschriebenen Instrumentenbehälters und Autoklaven sind in analoger Weise auch auf das vorgestellte Verfahren übertragbar und umgekehrt.

Die Erfindung wird nachfolgend anhand der in den Figuren dargestellten beispielhaften Ausführungsformen detaillierter erläutert. Es zeigen:
- Figur 1a: eine schematische Darstellung eines Autoklaven mit einem im Autoklaveninnenraum angeordneten Instrumentenbehälters gemäß einer ersten Ausführungsform;
- Figur 1b: eine schematische Darstellung eines Autoklaven mit einem im Autoklaveninnenraum angeordneten Instrumentenbehälters gemäß einer zweiten Ausführungsform;
- Figur 1c: eine schematische Darstellung eines Autoklaven mit einem im Autoklaveninnenraum angeordneten Instrumentenbehälters gemäß einer dritten Ausführungsform;
- Figur 1d: eine schematische Darstellung eines Autoklaven mit einem im Autoklaveninnenraum angeordneten Instrumentenbehälters gemäß einer vierten Ausführungsform;
- Figur 1e: eine Draufsicht auf einen Instrumentenbehälter gemäß der vierten Ausführungsform von Figur 1d;
- Figur 1f: eine schematische Darstellung eines Autoklaven mit einem im Autoklaveninnenraum angeordneten Instrumentenbehälters gemäß einer fünften Ausführungsform;
- Figur 1g: eine schematische Darstellung eines Autoklaven mit einem im Autoklaveninnenraum angeordneten Instrumentenbehälters gemäß einer sechsten Ausführungsform;
- Figur 2a: eine weitere Ausführungsform eines erfindungsgemäßen Instrumentenbehälters;
- Figur 2b: die Ausführungsform des Instrumentenbehälters der Figur 2a in geöffnetem Zustand;
- Figur 3: eine weitere Ausführungsform eines erfindungsgemäßen Instrumentenbehälters;
- Figur 4: die Ausführungsform des Instrumentenbehälters gemäß Figur 1d, in einer Variante des erfindungsgemäßen Verfahrens;
- Figur 5: einen Ausschnitt einer Ausführungsform des erfindungsgemäßen Instrumentenbehälters; und
- Figur 6: eine schematische Darstellung einer weiteren Variante des erfindungsgemäßen Verfahrens.

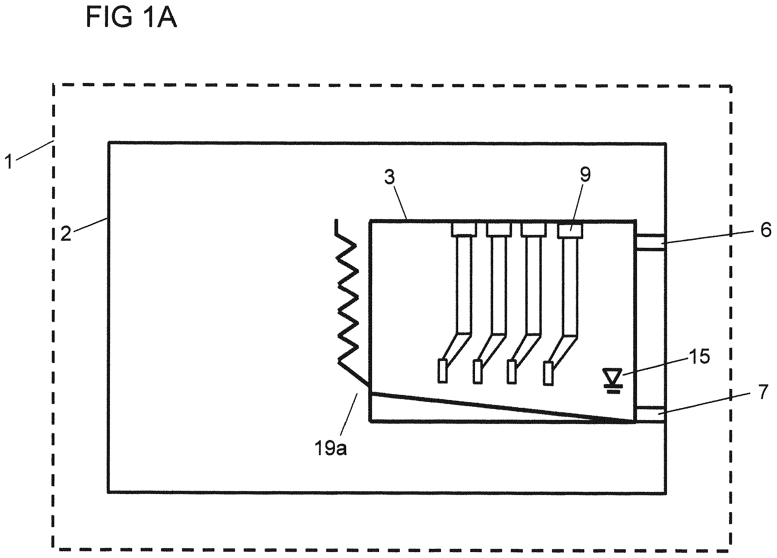

Figur 1a zeigt einen Autoklaven 1 als Vorrichtung zur hygienischen Aufbereitung medizinischer oder zahnmedizinischer Instrumente. Der Autoklav 1 weist einen Autoklaveninnenraum 2 und einen im Autoklaveninnenraum 2 angeordneten Instrumentenbehälter 3 auf.

Im Instrumentenbehälter 3 sind medizinische oder zahnmedizinische Instrumente 8 lösbar an einem geeigneten Trägersystem mit Adapter 9 zum Zwecke der Reinigung und Pflege angebracht. Der Instrumentenbehälter 3 ist in der vorliegenden Ausführungsform an einer Seitenwand des Autoklaveninnenraums 2 angeordnet und weist einen Anschluss 6 für die Zufuhr der Prozessmedien, wie Reinigungsflüssigkeit und Pflegeflüssigkeit in den Instrumentenbehälter 3 auf, wobei besagter Anschluss 6 im oberen Bereich des Instrumentenbehälters 3 angeordnet ist. Für die Abfuhr bzw. Ausfuhr der in den Instrumentenbehälter 3 eingeleiteten Flüssigkeit aus dem Instrumentenbehälter ist ein Anschluss 7 am Instrumentenbehälter 3 vorgesehen, der insbesondere im Bodenbereich 11 des Instrumentenbehälters zum vereinfachten Abführen der sich im Bodenbereich 11 des Instrumentenbehälters angesammelten Reinigungsflüssigkeit dient.

Der in Figur 1a dargestellte Instrumentenbehälter 3 weist eine offene Verbindung in Form einer labyrinthförmig gestalteten Leitung 19a zwischen der Autoklaveninnenraum 2 und dem Innenraum des Instrumentenbehälters 3 auf. Die Leitung 19a zur Zufuhr des Sterilisationsmediums verläuft parallel zu einer der Seitenflächen des Behälters 3 und ist somit vertikal im Autoklaveninnenraum 2 angeordnet. Die Leitung 19a basiert auf einem Labyrinthfilter mit mehreren Windungen, die einen stetig steigenden Verlauf, relativ zur Horizontalen, nehmen. Die Windungen erhöhen den Widerstand der Leitung gegenüber Fluiden.

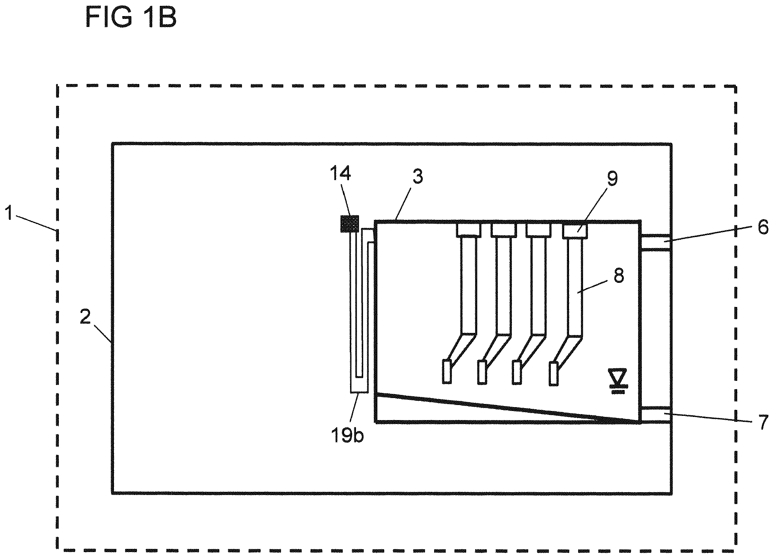

In der in Figur 1b gezeigten zweiten Ausführungsform ist die Zufuhrleitung 19b für das Sterilisationsmedium mäanderförmig gestaltet. Diese Gestaltungsform der Zufuhrleitung 19b basiert im Wesentlichen auf einer labyrinthförmigen Leitung mit stark reduzierter Windungsanzahl z.B. mit lediglich einer oder zwei Windungen. Die mäanderförmige Leitung 19b ist hierbei mit einem Filter 14 am oberen Ende versehen. Der Filter 14 bewirkt ein Auffangen von auftretenden Ölnebel. Der Filter 14 wird wiederum ebenfalls durch den Sterilisationsdampf ausgewaschen und regeneriert.

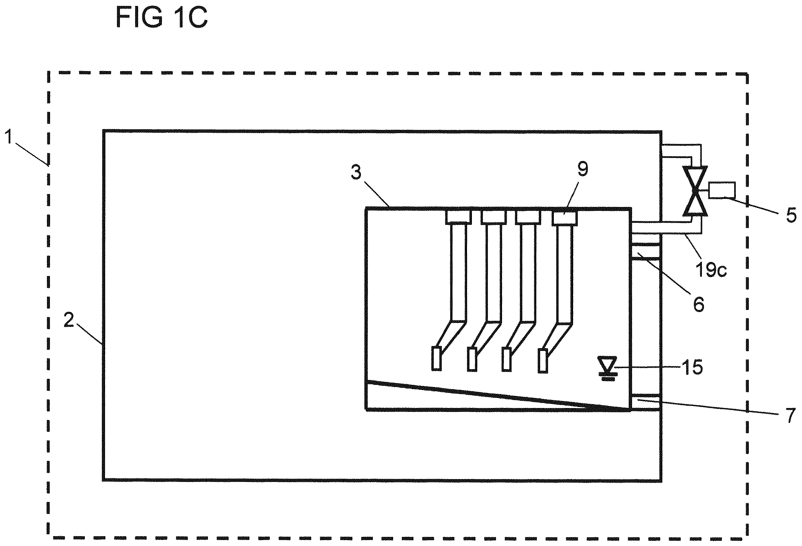

In der Ausführungsform in Figur 1c erfolgt die Öffnung des Systems für Sterilisationsmedien nicht auf ausschließlich konstruktivem, sondern zusätzlich auf prozesstechnischem Weg.

Konkret wird hier die Leitung für das Sterilisationsmedium nicht direkt aus der Kassette in die Autoklavenkammer geführt wie in den Ausführungsformen der Figuren 1a, 1b, sondern ist als Bypass gestaltet. Die Leitung 19c führt aus dem Autoklaveninnenraum 2 über die Kupplungsstelle für die Reinigungs- und Pflegemittel in den Instrumentenbehälter 3 hinein. Auf diese Weise wird ebenfalls eine Verbindung zwischen der Autoklavenkammer und dem Behälter geschaffen. Die Leitung 19c wird von einem Ventil 5, insbesondere einem elektronisch steuerbaren Ventil, unterbrochen. Ist das Ventil 5 geschlossen, so ist der Behälter hermetisch abgeriegelt und ein Durchgang von Reinigungsmitteln, Verunreinigungen und/ oder Ölnebeln ist nicht mehr möglich. Dementsprechend ist das Ventil 5 während dieser Prozessschritte geschlossen. Für eine Sterilisation kann das Ventil 5 geöffnet werden, wodurch ein Durchtritt des Sterilisationsmediums und ein gleichzeitiger Druckausgleich möglich werden.

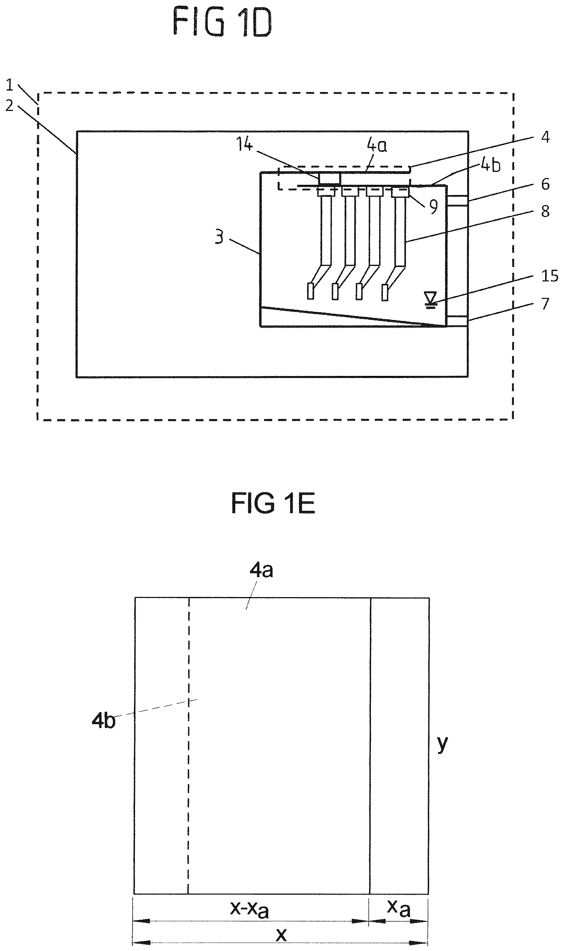

Gemäß der in Figur 1d gezeigten Ausführungsform kann im oberen Bereich auf der Oberseite des Instrumentenbehälters 3 eine mäanderförmig gestaltete Konstruktion 4 in Kombination mit einem Filter 14 vorgesehen sein, um ein Austreten von Flüssigkeit aus dem Instrumentenbehälter 3, welche insbesondere nach Reinigung und Pflege der darin befindlichen Instrumente mit Pflegemittelresten oder anderen zum Beispiel proteinhaltigen Rückständen versehen ist, zu vermeiden.

Die mäanderförmig gestaltete Konstruktion 4 ist im Falle der Ausführungsform der Figur 1d einstückig mit den Seitenflächen des Instrumentenbehälters 3 gestaltet. Zur Realisierung dieser spezifischen mäanderförmig gestalteten Konstruktion weisen die Seitenflächen des Instrumentenbehälters 3 jeweils unterschiedliche Längen auf bzw. Höhen auf. An den jeweiligen oberen Ende dieser Seitenflächen sind jeweils zwei Platten bzw. Abschnitte 4a,b senkrecht verlaufend zu den Seitenflächen angeordnet, wobei die Platten 4a,b mit den Seitenflächen einstückig ausgebildet sind. Diese Platten 4a, 4b liegen parallel dem Bodenbereich bzw. der Unterseite des Instrumentenbehälters 3 gegenüber und sind somit auch parallel zueinander angeordnet.

Die Platten bzw. Abschnitte 4a, 4b der mäanderförmigen Konstruktion weisen jeweils bevorzugt eine kleinere Oberfläche als die Bodenfläche des Instrumentenbehälters 3 auf. Mit anderen Worten, die Platten 4a, 4b sind in ihren Abmaßen bzw. Dimensionen kleiner im Vergleich zu den Bodenbereich des Instrumentenbehälters. Weist zum Beispiel der Bodenbereich des Instrumentenbehälters 3 eine bestimmte Länge x und eine bestimmte Breite y auf, so können die oberen Platten 4a, 4b eine gegenüber dem Bodenbereich um einen bestimmten Wert xₐ reduzierte Länge x-xₐ aufweisen, wobei die Breite y der Platten 4a, 4b gleich der Breite y des Bodenbereiches oder umgekehrt ist. Gleiche Annahmen gelten für die Dimensionen der Platte 4b. Eine derartige Konstruktion ist in Figur 1e dargestellt, welche eine Draufsicht auf die Oberseite eines Instrumentenbehälters 3 zeigt.

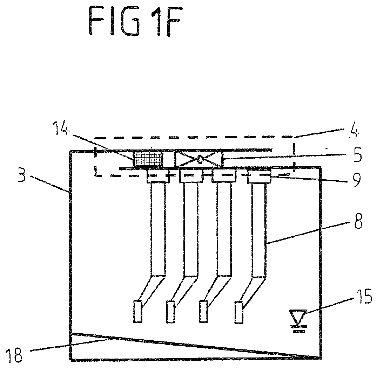

Die mäanderförmig gestaltete Konstruktion 4 kann gemäß der Ausführungsform der Figur 1f mit einem Filter 14 kombiniert sein, der gegenüber den Sterilisationsmedien, wie zum Beispiel Sattdampf durchlässig, jedoch Keime, Sporen, Viren und andere Mikroorganismen zurückhält. Die Verwendung eines Filters 14 ermöglicht somit die Verwendung des Instrumentenbehälters nach Reinigung, Pflege und Sterilisation zum sterilen Lagern der Instrumente außerhalb des Autoklavens. Der Filter 14 ist zwischen den aufgrund der unterschiedlichen Höhe der Seitenflächen beabstandeten Platten 4a, 4b über den gesamten Abstand zwischen diesen Platten angeordnet (d.h. der Filter 14 füllt diesen Abstand zwischen den Platten vollständig aus), so dass eine Filtration der in und aus dem Instrumentenbehälter 3 eingeführten Atmosphäre erfolgt. Es ist auch denkbar, dass zwischen den Platten 4a, b der mäanderförmig gestalteten Konstruktion 4 zusätzlich zum Sterilfilter 14 ein Ventil 5 angeordnet ist. Dieses Ventil 5 ist während der Reinigung und/oder Pflege geschlossen, wird jedoch während des Sterilisationsvorganges geöffnet.

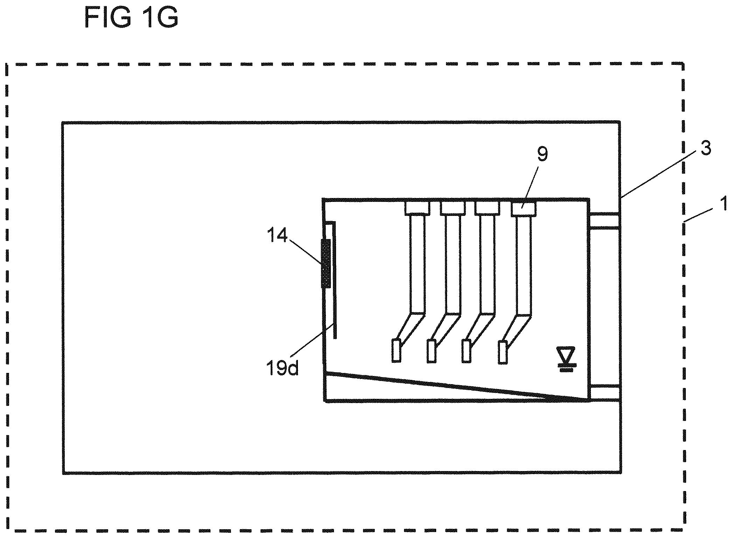

In der in Figur 1g gezeigten Ausführungsform weist der Behälter ein mäanderförmiges Bauteil 19d auf, das in einer Seitenwand des Behälters 3 angeordnet ist. In diesem Fall besteht das mäanderförmige Bauteil aus einem angewinkelten Materialteil aus einem längerem Schenkel und einen kürzerem Schenkel, die senkrecht zueinander angeordnet sind. Der längere Schenkel verläuft parallel zur Seitenwand und deckt eine in der Seitenwand vorgesehene Öffnung bzw. einen Durchbruch ab. Dabei deckt der längere Schenkel die Öffnung über dieselbige hinaus in der Weise ab, dass der Zugang des Sterilisationsmediums durch die Öffnung in den Behälterinnenraum gewährleistet ist. Das Sterilisationsmedium trifft bei Eintritt durch die Öffnung auf den länglichen Schenkel des Materialteiles und wird entlang diesem parallel zur Seitenwand des Behälters nach unten in den Behälter geleitet. Der kürzere Schenkel des Materialteiles bestimmt den Abstand des Materialteiles zur Seitenwand. In der Öffnung bzw. im Durchbrich der Seitenwand ist gemäß der Ausführungsform der Figur 1g ein Filter vorgesehen, der dem oben beschriebenen Filter 14 entspricht, vorgesehen. Auf diese Weise wird ein mäanderförmiger Zugang verwirklicht, die den Filter effektiv vor Verunreinigungen durch die kontaminierte Reinigungsflotte schützt.

Die medizinischen und zahnmedizinischen Instrumente sind, wie in den vorliegenden Figuren dargestellt, im Instrumentenbehälter 3 auf speziellen Adaptern 9 angebracht. Die Adapter 9 dienen einer Verbindung der medizinischen Instrumente mit einem Trägersystem bestehend aus einer Trägerplatte (nicht gezeigt). Die medizinischen oder zahnmedizinischen Instrumente werden auf die entsprechenden Adapter 9 jeweils aufgesteckt. Die Adapter 9 dienen des Weiteren der Zufuhr der Reinigungsflüssigkeit oder Pflegeflüssigkeit in die Innenkanäle der Instrumente 8. Derartige Adapter 9 sind dem Fachmann aus dem Stand der Technik bekannt.

Wie den Ausführungsformen des erfindungsgemäßen Instrumentenbehälters, dargestellt in den Figuren 1a bis f, zu entnehmen, weist der Instrumentenbehälter 3 ein Mittel 15 zur Einstellung bzw. Regulierung des Flüssigkeitsstandes der Reinigungsflüssigkeit 17 innerhalb des Instrumentenbehälters 3 auf. Das Mittel 15 zur Regulierung des Flüssigkeitspegels der als Trägermedium, wie später noch detaillierter beschrieben, verwendeten Reinigungsflüssigkeit 17 kann in verschiedenen Ausführungsformen verwendet werden, wie zum Beispiel in Form von geeigneten Dosierpumpen, Dosiervolumina oder auch Niveausensoren. Derartige Mittel sind dem Fachmann geläufig.

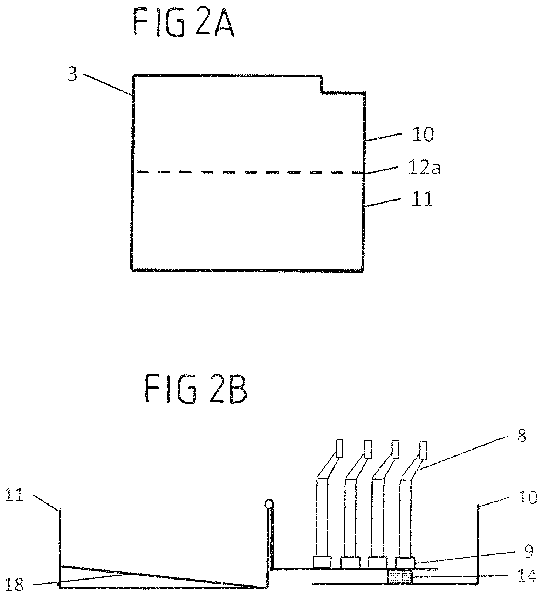

Figur 2a zeigt eine weitere bevorzugte Ausführungsform des Instrumentenbehälters 3. So kann der Instrumentenbehälter 3 aus zwei Hälften bestehen, die mittels eines geeigneten Scharniers miteinander beweglich und schwenkbar verbunden sind. Die Aufteilung des Instrumentenbehälters 3 bewirkt eine Unterteilung des Instrumentenbehälters in einen oberen Bereich 10, auch als Deckelbereich 10 bezeichnet, und einen unteren Bereich 11, auch als Bodenbereich 11 bezeichnet. Der Deckelbereich 10 ist über das Scharnier 12a beweglich und schwenkbar an dem Bodenbereich 11 befestigt, und stellt somit durch Aufklappen des Deckelbereiches einen Zugang zum Innenraum des Instrumentenbehälters 3 zur Bestückung des Instrumentenbehälters 3 mit den zu reinigenden Instrumenten 8 bereit.

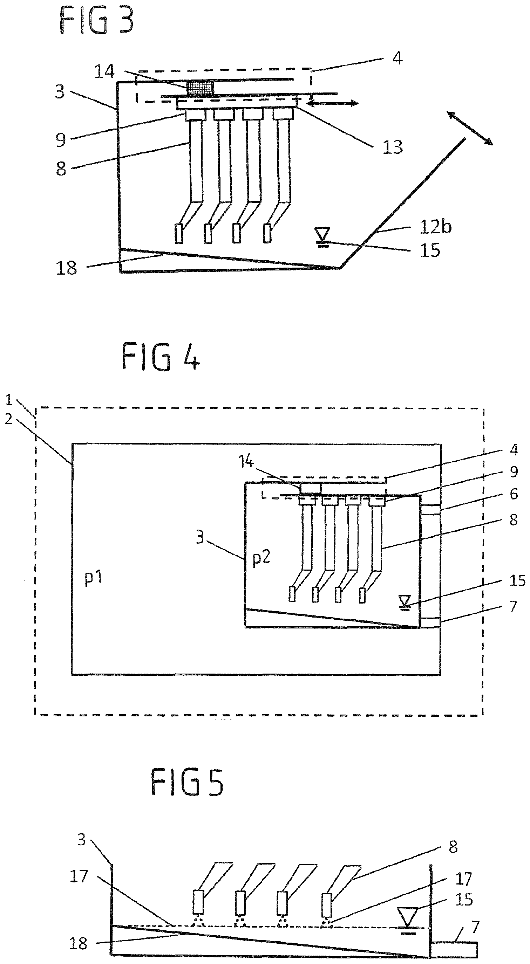

Neben der Unterteilung des Instrumentenbehälters 3 in einen oberen und einen unteren Bereich sind jedoch auch andere Ausführungsformen des Instrumentenbehälters denkbar. So ist es beispielsweise möglich, den Instrumentenbehälter 3 mit einer geeigneten Tür 12b auf einer Seite des Instrumentenbehälters 3 auszustatten. Dieser Türbereich 12b kann zum Beispiel gemäß der Ausführungsform dargestellt in Figur 3 derart gestaltet sein, dass eine Seitenfläche 12b des Instrumentenbehälters 3 in ihrer gesamten Länge entlang einer Seitenfläche des Instrumentenbehälters 3 beweglich an der Bodenplatte des Instrumentenbehälters 3 befestigt ist. Bei Aufklappen der als Türöffnung 12b zu bezeichnenden Seitenfläche des Instrumentenbehälters 3 ist somit ein seitlicher Zugang und damit eine seitliche Beladung des Instrumentenbehälters 3 mit den aufzubereitenden Instrumenten 8 möglich. In dieser Ausführungsform werden die Instrumente 8 auf einem Trägersystem 13, insbesondere einem Trägersystem in Leichtbauweise, in dem Instrumentenbehälter 3 befestigt.

Der Randbereich der als Beladungsöffnung fungierenden Tür 12b des Instrumentenbehälters ist mittels einer Dichtung verschließbar ausgelegt. Bei Verwendung eines abgedichteten Türbereiches sowie gleichzeitig der Verwendung eines mäanderförmigen Teiles als Spritzschutzelement 4, versehen mit einem Filter 14, der gegenüber Sterilisationsmedien durchlässig ist, jedoch Mikroorganismen, Sporen und Viren zurückhält, ist ein solcher Instrumentenbehälter 3 gleichermaßen zum sterilen Lagern der Instrumente 8 nach Beendigung der hygienischen Aufbereitung und Sterilisation geeignet, wobei die verschiedenen Anschlüsse des Instrumentenbehälters entsprechend verschlossen sind.

Bezug nehmend auf die in Figur 4 dargestellte Ausführungsform des Instrumentenbehälters 3 wird ein mögliches Verfahren zur hygienischen Aufbereitung der im Instrumentenbehälter 3 angeordneten Instrumente 8 beschrieben. Gemäß der in Figur 4 gezeigten Ausführungsform umfasst der Instrumentenbehälter 3 ein am Boden des Behälters 3 angebrachtes Anschlussstück 7, das sowohl zum Ankuppeln einer verschließbaren Entleerungsleitung vorgesehen ist als auch bei Ausführung zum Beispiel in Form eines Saugrüssels als Abströmventil dienen kann. Der Bodenbereich 11 des Instrumentenbehälters 3 ist gegenüber den Reinigungs- und/oder Pflegemedien bzw. Flüssigkeiten abdichtend auszulegen. Für den Fall, dass das druckausgleichende und den Flüssigkeitsaustritt verhindernde Mittel 4 als Kombination eines mäanderförmigen Teiles mit gegebenenfalls einem geeigneten Sterilisationsfilter ausgebildet ist, und somit also nicht über ein Abschottungsventil 5 verfügt, sind zum Abfangen von bei der Ölpflege von zahnmedizinischen Instrumenten 8 auftretenden Aerosolen zusätzliche verfahrenstechnische Schritte notwendig. Hierbei wird das Austreten von Aerosolen verfahrenstechnisch durch Anlegen einer Druckdifferenz Δ p zwischen der Autoklavenkammer 2 mit dem Druck p1 und dem Innenraum des Instrumentenbehälters 3 mit dem Druck p2 verhindert. Die Druckdifferenz sollte dabei derart sein, dass p1 größer p2 ist, so dass in der Autoklavenkammer 2 ein höherer Druck p1 herrscht als in dem Instrumentenbehälter 3 mit dem Druck p2. Auf diese Weise wird eine Strömung, zum Beispiel eine Strömung des Sattdampfes, aus der Autoklavenkammer 2 in den Innenraum des Instrumentenbehälters 3 erzeugt, welche eine Sperre für eventuell austretende Aerosole bildet. Konkret erfolgt das Erzeugen der gewünschten Druckdifferenz durch Anlegen eines Überdrucks p1 im Autoklaveninnenraum, bei einem gleichzeitig geöffneten Abströmventil 7 im unteren Bodenbereich des Instrumentenbehälters 3.

Gemäß dem vorliegenden Verfahren ist es vorgesehen, die zum Einsatz kommende Reinigungsflüssigkeit im Bodenbereich des Instrumentenbehälters 3 mit einem bestimmten Flüssigkeitsstand bzw. Pegelstand vorzulegen. Die Einstellung des Pegelstandes der Reinigungsflüssigkeit erfolgt durch das Regulierungsmittel 15. Wie in Figur 5 gezeigt, dient die am Boden des Instrumentenbehälters gesammelte Reinigungsflüssigkeit als Trägermedium 17 für die aus in dem Instrumentenbehälter 3 angeordneten Instrumenten 8 ausgeblasene ölhaltige Pflegeflüssigkeit 16. Das Auffangen der ölhaltigen Pflegeflüssigkeit 16 im Trägermedium 17 ermöglicht die Entfernung der ölhaltigen Pflegeflüssigkeit 16 nach deren Verwendung aus dem Instrumentenbehälter 3 durch eine einfache Entleerung des Instrumentenbehälters 3 über das Anschlussstück 7 aus dem Instrumentenbehälter 3. Die Reinigungsflüssigkeit 17 wird entweder für den Zweck der Ölaufnahme in den Instrumentenbehälter 3 eingeleitet oder wird als Produkt der vorhergegangenen Instrumentenaufbereitung aufgefangen und im Bodenbereich entsprechend gesammelt. Das Auffangen des ölhaltigen Pflegemittels 16 in der als Trägermedium fungierenden Reinigungsflüssigkeit 17 ermöglicht somit einen Abtransport sämtlicher Verunreinigungen und Pflegemittelreste aus dem Instrumentenbehälter 3 zusammen mit der Reinigungsflüssigkeit, so dass eine Kontamination des Autoklaveninnenraums 2 zu einem späteren Zeitpunkt der Aufbereitung demnach ausgeschlossen ist.

Um den Abtransport der mit der ölhaltigen Pflegeflüssigkeit 16 versehenen Reinigungsflüssigkeit 17 aus dem Instrumentbehälter 3 zu erleichtern, ist es zweckmäßig, eine schräge Ebene 18 in den Bodenbereich des Instrumentenbehälters 3 vorzusehen. Diese schräge Ebene 18 ist dabei so konstruiert, dass ein Gefälle in Richtung des Abführanschlusses 7 zur Entleerung des Instrumentenbehälters 3 entsteht; siehe hierzu auch Figur 5.

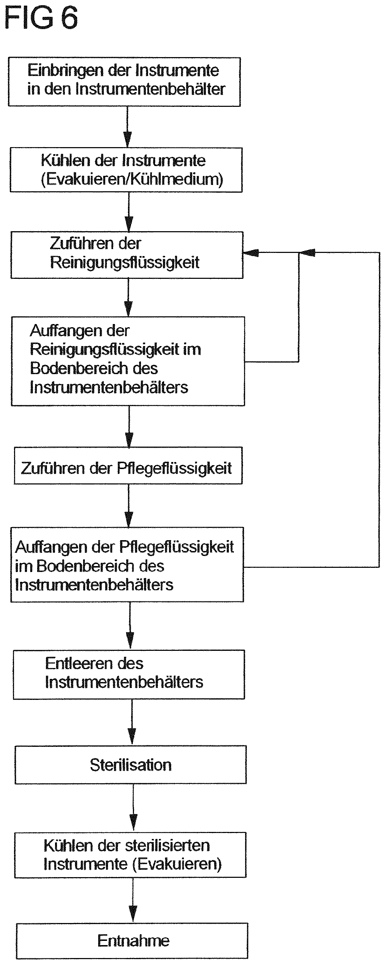

Der Ablauf einer Ausführungsform eines Verfahrens zur hygienischen Aufbereitung von medizinischen und zahnmedizinischen Instrumenten unter Verwendung eines erfindungsgemäßen Instrumentenbehälters 3 ist im Folgenden unter Bezugnahme auf die schematische Darstellung der Verfahrensschritte gemäß Figur 6 beschrieben. Entsprechend werden die zu reinigenden Instrumente 8 zunächst unter Verwendung eines Trägersystems 13 mit entsprechenden Adaptern 9 im Instrumentenbehälter befestigt. Eine Möglichkeit des Beladens des Instrumentenbehälters 3 besteht in einem Einschieben des Trägersystems 13 mit den daran befestigten Instrumenten 8 durch eine seitliche Türöffnung, die in Form einer beweglichen und schwenkbaren Seitenwand des Instrumentenbehälters 3 ausgelegt sein kann (siehe hierzu auch Figur 3).

Nach Einbringen der aufzubereitenden Instrumente 8 in den Instrumentenbehälter 3 wird der Autoklaveninnenraum 2 evakuiert. Die Evakuierung kann durch Druckreduzierung auf einen Druck von weniger als 500 mbar erfolgen. Der durch die Evakuierung verringerte Druck im Autoklaveninnenraum 2 reduziert die Zeit bis zur Erwärmung der Instrumente, zum Beispiel ist es so möglich, die Erwärmungszeit der Instrumente auf die Koagulationstemperatur von Proteinen, die zum Beispiel im Blut enthalten sind, um bis zu 64% zu erhöhen. Eine derartige Zeitverzögerung der Temperaturerwärmung der Instrumente ist insbesondere dann gewünscht, wenn sich der Start der hygienischen Aufbereitung verzögert und somit nicht unmittelbar nach dem Einbringen der Instrumente 8 in den Instrumentenbehälter 3 und in den Autoklaveninnenraum 2 erfolgt. Zu Beginn des sich anschließenden hygienischen Aufbereitungsprozesses erfolgt ein Druckausgleich, um das Einbringen der Reinigungsflüssigkeit 17 und der Pflegeflüssigkeit 16 in den Instrumentenbehälter 3 zu ermöglichen. Eine weitere Möglichkeit der Kühlung der Instrumente 8 im Falle eines verzögerten Starts der hygienischen Aufbereitung besteht in der aktiven Kühlung der Instrumente 8 durch Verwendung eines Kühlemediums auf eine Temperatur unterhalb der Koagulationstemperatur von Blut. Als geeignetes Kühlmedium kann zum Beispiel Wasser verwendet werden.

Der sich an die Kühlung der Instrumente und gegebenenfalls an den gegebenenfalls erforderlichen Druckausgleich zwischen Autoklaveninnenraum 2 und Instrumentenbehälter 3 anschließende Schritt umfasst das Zuführen von geeigneter Reinigungsflüssigkeit 17, bei der es sich bevorzugterweise um reines Wasser handelt, das gegebenenfalls entgast sein kann. Eine Entgasung erhöht die Aufnahmefähigkeit des Wassers für Öle. Es ist auch denkbar, das Wasser mit waschaktiven Substanzen zur Erhöhung der Aufnahmefähigkeit des Wassers für hydrophobe Substanzen aus der ölhaltigen Pflegeflüssigkeit 17 zu versetzen.

Nach äußerem und innerem Reinigen der im Instrumentenbehälter 3 angebrachten Instrumente 8 mittels der zugeführten Reinigungsflüssigkeit 17 wird diese im Bodenbereich des Instrumentenbehälters 3 aufgefangen und erneut zum Reinigen der Instrumente 8 verwendet. Somit erfolgt die Reinigung der Instrumente 8 bevorzugterweise durch ein Umwälzen der Reinigungsflüssigkeit 17. Diese ist besonders vorteilhaft, wenn das Umwälzen auf verschiedenen Temperaturniveaus erfolgt, wobei zunächst eine kalte Vorreinigung bei einer Temperatur unter 45°C erfolgt und anschließend eine Reinigung mit einer erwärmten Reinigungsflüssigkeit 17.

Gemäß dem in Figur 6 angeführten Verfahrensschema verbleibt zumindest ein Teil der Reinigungsflüssigkeit 17 nach der Warmreinigung im Instrumentenbehälter 3 im Bodenbereich desselbigen und dient als Auffangmedium bzw. Trägermedium zum Auffangen von ölhaltiger Pflegeflüssigkeit 16, die in dem sich an den Reinigungsschritt anschließenden Pflegeschritt verwendet wird. In diesem Pflegeschritt wird jedem Instrument 8 eine bestimmte Menge Öl in Form einer ölhaltigen Pflegereinigung zudosiert und das überschüssige Öl wird anschließend mit Druckluft ausgeblasen und von dem sich im Bodenbereich des Instrumentenbehälters 3 befindlichen Trägermedium 17 aus Reinigungsflüssigkeit aufgenommen. Es ist dabei generell vorstellbar, dass ein Umwälzen der mit dem ausgeblasenen ölhaltigen Pflegemittel versehenen Reinigungsflüssigkeit zum Zwecke der Minderung der Aerosolbildung erfolgt.

Nach erfolgter Reinigung und Pflege wird die mit ölhaltigen Pflegemittelresten versehene Reinigungsflüssigkeit 17 aus dem Instrumentenbehälter 3 über ein entsprechendes Anschlussstück 7 entleert. Eine entsprechende Entleerung des Instrumentenbehälters 3 kann insbesondere dann effektiv erfolgen, wenn der Transport der Flüssigkeit unter Verwendung einer Pumpe und der damit erzeugten Sogwirkung beschleunigt wird. Eine andere Möglichkeit besteht in der Beaufschlagung des Instrumentenbehälters 3 mit Druckluft, um das Entleeren des Instrumentenbehälters 3 vor der sich daran anschließenden Sterilisation zu beschleunigen.

Die nachfolgende Sterilisation wird mit den aus dem Stand der Technik bekannten Mitteln durchgeführt. Insbesondere wird Sattdampf in den Autoklaveninnenraum 2 und in den sich darin befindlichen Instrumentenbehälter 3 zum Zwecke der Sterilisation der Instrumente eingeführt.

In Analogie zu dem vor der hygienischen Aufbereitung durchzuführenden Schritt des Abkühlens der Instrumente 8 im Instrumentenbehälter 3 wird nach Abschluss der Sterilisation ein entsprechender Abkühlschritt eingeführt, der bevorzugt die Evakuierung des Autoklaveninnenraums 2 vorsieht. Auf diese Weise wird einer Schädigung der Instrumente 8 durch die im Autoklaveninnenraum 2 nach Sterilisation eingestellten hohen Temperaturen vorgebeugt. Nach Abkühlung der Instrumente 8 erfolgt eine Entnahme der gereinigten Instrumente 8 aus dem Instrumentenbehälter 3 zur unmittelbaren Verwendung. Ist eine direkte Weiterverwendung der gereinigten Instrumente nicht vorgesehen, so können diese in dem Instrumentenbehälter 3 bis zu deren Einsatz im Instrumentenbehälter 3 steril gelagert werden.

### Bezugszeichenliste

- 1: Autoklav
- 2: Autoklaveninnenraum
- 3: Instrumentenbehälter
- 4a, b: Platten der starren mäanderförmigen Konstruktion
- 5: Ventil
- 6: Anschluss für Flüssigkeitszufuhr in den Instrumentenbehälter 3
- 7: Anschluss zur Flüssigkeitsentleerung des Instrumentenbehälters 3
- 8: medizinische / zahnmedizinische Instrumente
- 9: Adapter
- 10: oberer Bereich des Instrumentenbehälters 3
- 11: unterer Bereich des Instrumentenbehälters 3
- 12a: Scharnier zwischen oberen und unteren Bereich 10, 11
- 12b: seitliche Türöffnung
- 13: Trägersystem für Instrumente 8
- 14: Filter
- 15: Regulierungsmittel
- 16: Pflegeflüssigkeit
- 17: Reinigungsflüssigkeit
- 18: Transportmittel
- 19a: labyrinthförmige Zufuhrleitung für das Sterilisationsmedium
- 19b: mäanderförmige Zufuhrleitung für das Sterilisationsmedium
- 19c: Bypass zur Zufuhr des Sterilisationsmediums
- 19d: mäanderförmiges Bauteil zur Zufuhr des Sterilisationsmediums

## Patentansprüche

1. Behälter (3) zur Aufbereitung von medizinischen oder zahnmedizinischen Instrumenten in einem Autoklaven (1) und zum lösbaren Anbringen in einem Innenraum (2) eines Autoklaven (1), umfassend
- mindestens einen ersten Anschluss (6) zur Zufuhr von Flüssigkeiten zur äußeren und inneren Reinigung der medizinischen oder zahnmedizinischen Instrumente (8) in den Behälter (3),
- mindestens einen zweiten Anschluss (7) zur Abfuhr der Flüssigkeiten zur äußeren und inneren Reinigung der medizinischen oder zahnmedizinischen Instrumente (8) und/oder dem Sterilisationsmedium aus dem Behälter (3),
wobei der erste Anschluss (6) und der zweite Anschluss (7) des Behälters (3) jeweils mit mindestens einem Anschluss des Autoklaven (1) zur Zufuhr der Reinigungsflüssigkeiten in den Behälter (3) und mit mindestens einem Anschluss des Autoklaven (1) zur Abfuhr der Reinigungsflüssigkeiten aus dem Behälter (3) verbindbar sind, ohne das die Flüssigkeiten zur äußeren und inneren Reinigung der medizinischen oder zahnmedizinischen Instrumente (8) in den Innenraum des Autoklaven (1) austreten;
**gekennzeichnet durch**
- mindestens eine Zufuhrleitung (4, 19a, 19b) zur Zufuhr von mindestens einem Sterilisationsmedium aus dem Innenraum (2) des Autoklaven, in dem der Behälter (3) angebracht ist, in den Behälter (3), wobei die erste Zufuhrleitung (4, 19a, 19b) zur Zufuhr des Sterilisationsmediums in den Behälter (3) als labyrinthförmige Leitung (19a) oder als maänderförmige Leitung (19b) ausgebildet ist,
wobei die mindestens eine Zufuhrleitung (4, 19a, 19b) von dem mindestens einem Sterilisationsmedium passiert werden kann, jedoch für die Flüssigkeiten zur äußeren und inneren Reinigung der medizinischen oder zahnmedizinischen Instrumente (8) nicht passierbar sind,
- wobei der Behälter (3) als nicht selbsttragend gegenüber Prozessdrücken einer Sattdampfsterilisation ausgelegt ist.

2. Behälter nach Anspruch 1, **dadurch gekennzeichnet, dass** mindestens eine zusätzliche Zufuhrleitung (19c) zur Zufuhr des Sterilisationsmediums vom Behälter (3) aus dem Autoklaveninnenraum (2) vorgesehen ist, wobei diese zusätzliche Zufuhrleitung (19c) zunächst aus dem Autoklaveninnenraum (2) hinausführt und wieder in den Autoklaveninnenraum (2) in Form eines Bypass zurückgeführt wird.

3. Behälter nach einem der vorhergehenden Ansprüche , **dadurch gekennzeichnet, dass** die mäanderförmige Leitung (19b) in Form eines Schlauches oder Rohres und/oder in Form eines starren mäanderförmigen Teiles an einer Seitenfläche des Behälters (3) und/oder auf der Oberseite des Behälters (3) ausgebildet ist.

4. Behälter nach einem der vorhergehenden Anschlüsse, **gekennzeichnet durch** mindestens ein in dem Behälter (3) lösbar angeordnetes Trägersystem (13) aus Trägerplatte mit daran ausgebildeten Adaptern (9) zu Aufnahme der medizinischen oder zahnmedizinischen Instrumente.

5. Behälter nach einem der vorhergehenden Ansprüche, **dadurch gekennzeichnet, dass** der Behälter (3) mindestens zwei miteinander verbundene Teile (10, 11) umfasst, die den Behälter in einen unteren Bereich und einen oberen Bereich aufteilen.

6. Behälter nach einem der vorhergehenden Ansprüche, **gekennzeichnet durch** mindestens eine im Bodenbereich des Behälters (3) vorgesehenes Gefälle in Form einer schiefen Ebene (18) zum Transport der im Instrumentenbehälter (3) enthaltenen Flüssigkeit (17) in Richtung des Anschlusses (7) zur Abfuhr der Flüssigkeit aus dem Behälter (3).

7. Behälter nach einem der vorhergehenden Ansprüche, **gekennzeichnet durch** mindestens ein Regulierungsmittel (15) zur Einstellung des Flüssigkeitsstandes der Flüssigkeit (17) innerhalb des Instrumentenbehälters (3).

8. Behälter nach einem der vorhergehenden Ansprüche, **dadurch gekennzeichnet, dass** der Instrumentenbehälter (3) aus einem ein Material mit einer Wärme-Festigkeits-Charakteristik von kleiner als 2*10⁻⁶ N/s·K² hergestellt ist.

9. Behälter nach einem der vorhergehenden Ansprüche, **dadurch gekennzeichnet, dass** der erste Anschluss (6) und der zweite Anschluss (7) des Behälters (3) jeweils über eine trennbare Verbindungsleitung mit dem Anschluss des Autoklaven (1) zur Zufuhr von Flüssigkeiten in den Behälter (3) und dem Anschluss des Autoklaven (1) zur Abfuhr von Flüssigkeiten aus dem Behälter (3) verbindbar sind.

## Claims

1. A container (3) for the treatment of medical or dental instruments in an autoclave (1) and for the releasable attachment in an interior space (2) of an autoclave (1), comprising
- at least one first port (6) for the supply of liquids for the outer and inner cleaning of the medical or dental instruments (8) into the container (3),
- at least one second port (7) for the discharge of the liquids for the outer and inner cleaning of the medical or dental instruments (8) and/or the sterilization medium from the container (3),
wherein the first port (6) and the second port (7) of the container (3) each are connectable to at least one port of the autoclave (1) for the supply of the cleaning liquids into the container (3) and to at least one port of the autoclave (1) for the discharge of the cleaning liquids from the container (3), without the liquids for the outer and inner cleaning of the medical or dental instruments (8) exiting into the interior space of the autoclave (1);
**characterized by**
- at least one supply line (4, 19a, 19b) for the supply of at least one sterilization medium from the interior space (2) of the autoclave, in which the container (3) is mounted, into the container (3), wherein the first supply line (4, 19a, 19b) for the supply of the sterilization medium into the container (3) is configured as a labyrinth-shaped line (19a) or as a meander-shaped line (19b),
wherein the at least one supply line (4, 19a, 19b) can be passed by the at least one sterilization medium, but are not passable for the liquids for the outer and inner cleaning of the medical or dental instruments (8),
wherein the container (3) is designed to be not self-supporting with respect to process pressures of a saturated steam sterilization.

2. The container according to claim 1, **characterized in that** at least one additional supply line (19c) is provided for the supply of the sterilization medium from the container (3) out of the autoclave interior space (2), wherein this additional supply line (19c) first leads out of the autoclave interior space (2) and is again recirculated into the autoclave interior space (2) in the form of a bypass.

3. The container according to any of the preceding claims, **characterized in that** the meander-shaped line (19b) is configured in the form of a hose or tube and/or in the form of a rigid meander-shaped part on a side face of the container (3) and/or on the upper side of the container (3).

4. The container according to any of the preceding claims, **characterized by** at least one carrier system (13) releasably arranged in the container (3), comprising a carrier plate with adapters (9) formed thereon for receiving the medical or dental instruments.

5. The container according to any of the preceding claims, **characterized in that** the container (3) comprises at least two parts (10, 11) connected to each other, which divide the container into a lower region and an upper region.

6. The container according to any of the preceding claims, **characterized by** at least one slope in the form of an inclined plane provided in the bottom region of the container (3) for the transport of the liquid (17) contained in the instrument container (3) in the direction of the port (7) for the discharge of the liquid from the container (3).

7. The container according to any of the preceding claims, **characterized by** at least one regulating means (15) for adjusting the liquid level of the liquid (17) within the instrument container (3).

8. The container according to any of the preceding claims, **characterized in that** the instrument container (3) is made of a material with a heat resistance characteristic of less than 2*10⁻⁶ N/s·K².

9. The container according to any of the preceding claims, **characterized in that** the first port (6) and the second port (7) of the container (3) each are connectable to the port of the autoclave (1) for the supply of liquids into the container (3) and to the port of the autoclave (1) for the discharge of liquids from the container (3) via a separable connecting line.

## Revendications

1. Récipient (3) pour la préparation d'instruments médicaux ou dentaires dans un autoclave (1) et pour la mise en place amovible dans un espace intérieur (2) d'un autoclave (1), comprenant
- au moins un premier raccord (6) pour l'acheminement de fluides pour le nettoyage extérieur et intérieur des instruments médicaux ou dentaires (8) dans le récipient (3),
- au moins un deuxième raccord (7) pour l'évacuation des fluides pour le nettoyage extérieur et intérieur des instruments médicaux ou dentaires (8) et/ou du milieu de stérilisation hors du récipient (3),
dans lequel le premier raccord (6) et le deuxième raccord (7) du récipient (3) peuvent être reliés respectivement à au moins un raccord de l'autoclave (1) pour l'acheminement des fluides de nettoyage dans le récipient (3) et à au moins un raccord de l'autoclave (1) pour l'évacuation des fluides de nettoyage hors du récipient (3) sans que les fluides pour le nettoyage extérieur et intérieur des instruments médicaux ou dentaires (8) sortent dans l'espace intérieur de l'autoclave (1) ;
**caractérisé par**
- au moins une conduite d'acheminement (4, 19a, 19b) pour l'acheminement d'au moins un milieu de stérilisation hors de l'espace intérieur (2) de l'autoclave, dans lequel le récipient (3) est placé, dans le récipient (3), dans lequel la première conduite d'acheminement (4, 19a, 19b) pour l'acheminement du milieu de stérilisation dans le récipient (3) est réalisée en tant que conduite en forme de labyrinthe (19a) ou en tant que conduite en forme de méandres (19b),
dans lequel l'au moins une conduite d'acheminement (4, 19a, 19b) peut être parcourue par l'au moins un milieu de stérilisation, mais ne peut pas être parcourue par les fluides pour le nettoyage extérieur et intérieur des instruments médicaux ou dentaires (8),
- dans lequel le récipient (3) est conçu comme étant non autoportant par rapport aux pressions de processus d'une stérilisation à vapeur saturée.

2. Récipient selon la revendication 1, **caractérisé en ce qu'**au moins une conduite d'acheminement supplémentaire (19c) pour l'acheminement du milieu de stérilisation du récipient (3) hors de l'espace intérieur de l'autoclave (2) est prévue, dans lequel cette conduite d'acheminement supplémentaire (19c) sort tout d'abord de l'espace intérieur d'autoclave (2) et revient dans l'espace intérieur d'autoclave (2) sous la forme d'une dérivation.

3. Récipient selon l'une quelconque des revendications précédentes, **caractérisé en ce que** la conduite en forme de méandres (19b) est réalisée sous la forme d'un tuyau ou d'un tube et/ou sous la forme d'une partie rigide en forme de méandres au niveau d'une surface latérale du récipient (3) et/ou sur le côté supérieur du récipient (3).

4. Récipient selon l'une quelconque des revendications précédentes, **caractérisé par** au moins un système de support (13) disposé de manière amovible dans le récipient (3) de la plaque de support avec des adaptateurs (9) réalisés au niveau de celle-ci pour la réception des instruments médicaux ou dentaires.

5. Récipient selon l'une quelconque des revendications précédentes, **caractérisé en ce que** le récipient (3) comprend au moins deux parties (10, 11) reliées l'une à l'autre qui séparent le récipient en une zone inférieure et une zone supérieure.

6. Récipient selon l'une quelconque des revendications précédentes, **caractérisé par** au moins une pente prévue dans la zone de fond du récipient (3) sous la forme d'un plan oblique (18) pour le transport du fluide (17) contenu dans le récipient d'instruments (3) en direction du raccord (7) pour l'évacuation du fluide hors du récipient (3).

7. Récipient selon l'une quelconque des revendications précédentes, **caractérisé par** au moins un moyen de régulation (15) pour le réglage du niveau de fluide du fluide (17) à l'intérieur du récipient d'instruments (3).

8. Récipient selon l'une quelconque des revendications précédentes, **caractérisé en ce que** le récipient d'instruments (3) est fabriqué en un matériau avec une caractéristique de résistance à la chaleur inférieure à 2*10⁻⁶ N/s·K².

9. Récipient selon l'une quelconque des revendications précédentes, **caractérisé en ce que** le premier raccord (6) et le deuxième raccord (7) du récipient (3) peuvent être reliés respectivement par l'intermédiaire d'une conduite de liaison séparable avec le raccord de l'autoclave (1) pour l'acheminement de fluides dans le récipient (3) et le raccord de l'autoclave (1) pour l'évacuation de fluides hors du récipient (3).
